# EUROPEAN PATENT APPLICATION

(11) **EP 2 465 498 A1**
(43) Date of publication of application: **20.06.2012**
(21) Application number: 10382314.2
(22) Date of filing: 23.11.2010
(51) Int. Cl.: A61K 31/137, A61K 31/341, A61K 31/381, A61K 31/4184, A61K 31/4402, A61K 31/519, A61K 31/52, C07C 211/27, C07C 211/29, C07C 217/58, C07C 255/58, C07D 233/58, C07D 235/08, C07D 473/16, C07D 473/34, C07D 473/40, C07D 487/22

(54) **Diphenyl-amine derivatives: uses, process of synthesis and pharmaceutical compositions**

(71) Applicant: FAES FARMA, S.A., 48940 Lamiako - Leioa - Vizcaya (ES)
(72) Inventor: RODES SOLANES, Rosa, E-48940, Lamiako (Lejona) - Vizcaya (ES); GARCÍA DOMÍNGUEZ, Neftalí, E-48940, Lamiako (Lejona) - Vizcaya (ES); LÓPEZ ORTEGA, Beatriz, E-48940, Lamiako (Lejona) - Vizcaya (ES); ÁLVAREZ DE MON SOTO, Melchor, E-48940, Lamiako (Lejona) - Vizcaya (ES); DE LA HERA MARTÍNEZ, Antonio, E-48940, Lamiako (Lejona) - Vizcaya (ES); MUÑOZ MUÑOZ, Ana, E-48940, Lamiako (Lejona) - Vizcaya (ES); LEDO GÓMEZ, Francisco, E-48940, Lamiako (Lejona) - Vizcaya (ES)
(74) Representative: ABG Patentes, S.L.

(57) **Abstract**

The invention relates to compounds of formula (I): 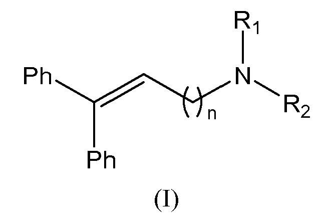 or a pharmaceutically acceptable salt, prodrug or solvate thereof, a method of synthesis of said compounds, pharmaceutical compositions comprising them and their use as a medicament for treating inflammatory diseases.

## Description

### FIELD OF THE INVENTION

The present invention relates to diphenyl-derivatives, method of synthesis, compositions comprising them and their use in the preparation of a medicament for immune-modulatory therapy (for example, immune diseases or certain types of cancer).

### STATE OF THE ART

Inflammation is a complex immune system reaction innate to vascularized tissues consisting of the accumulation and activation of leukocytes and of plasma proteins at a site of infection, exposure to toxins or cell injury.

The inflammation begins with changes in the blood vessels which promote leukocyte recruitment.

Local adaptive immune responses can stimulate inflammation. Although this has a protective effect on controlling infections and enhancing tissue repair, it can also cause tissue injury and disease.

The so-called immune inflammation is a consequence of an adaptive immune response to the antigen. The cell infiltrate at the inflammation site can contain cells of the innate immune system, such as neutrophils and macrophages, which are recruited as a consequence of the actions of the cytokines produced by T cells.

Cytokines are a family of proteins which mediate many innate immunity and adaptive immunity responses. The same cytokines can be produced by many types of cells, and one and the same cytokine often acts on different cell types.

Cytokines are synthesized as a response to inflammatory or antigenic stimuli and they normally act locally in an autocrine or paracrine manner, binding to high affinity receptors present in the target cells. Certain cytokines can be produced in sufficient quantities to circulate and exert endocrine actions. Cytokines further act as growth factors for many cell types.

Cytokines mediate their actions through a high affinity binding to receptors which belong to a limited number of structural families. Different cytokines use specialized signaling pathways, such as the JAK/STAT pathway.

Cytokines mediating innate immunity are mainly produced by activated microphages and include: TNF and IL-1 as mediators of acute inflammatory reactions to microorganisms; chemokines as leukocyte recruiters at the foci of inflammation; IL-12 as a macrophage-activating cytokine (IFN-gamma) production stimulant; type I interferons as antiviral cytokines, and IL-10 as a macrophage inhibitor.

Cytokines mediating and regulating the activation and effector phases of adaptive immunity are mainly produced by antigen-stimulated T-cells and include: IL-2 as the main T-cell growth factor; IL-4 as a stimulant of IgE synthesis and the development of Th2 cells from collaborating virgin T-cells; IL-5 as an eosinophil activator; IFN-gamma as a macrophage activator, and TGF-beta as a T-cell proliferation and leukocyte activation inhibitor.

CD4+ collaborating T-cells can differentiate into specialized Th1 effector cells, which secrete IFN-gamma, which favors phagocyte-mediated immunity, or into Th2 cells, which secrete IL-4 and IL-5, which favor IgE-, eosinophil- and mastocyte-mediated immunity.

In summary, cytokines perform many functions which are critical for defending the host against pathogens and they provide a nexus of union between innate and adaptive immunity. Cytokines also regulate the magnitude and the nature of immune responses, affecting lymphocyte growth and differentiation. Finally, cytokines provide important amplification mechanisms which allow a small number of lymphocytes specific for any one antigen to activate various effector mechanisms to eliminate the antigen. An excessive production or action of the cytokines may have pathological consequences.

The administration of cytokines, soluble receptors or inhibitors of cytokines has become, at present, a novel and effective approach for modifying biological responses associated with both acute and chronic immune and inflammatory diseases, and also for the treatment of many types of cancer. The possibility of treating patients with cancer by means of immune strategies has given hope to immunologists and biologists of cancer. The main reason for this interest is based on the fact that current cancer therapies depend on drugs which destroy dividing cells or block cell division, and these treatments have serious effects on normal proliferating cells in cancer patients. Cancer treatment therefore causes significant morbidity and mortality. Unlike these treatments, immune-modulatory therapy has the potential to be a treatment with the highest specificity and lowest toxicity that can be imagined.

However, virtually all of these new therapeutic tools have a biological origin and are highly specific for a single cytokine. This involves a series of tolerance risks, and the risks of generating immune reactions and induction reactions involving a considerable imbalance in the complex immune system.

The series of compounds presented in this invention, in addition to being small molecules and not biological compounds, have the particularity of acting clearly on more than one related cytokine but not on others, potentially acting as modulators more than strict inhibitors, without modifying cell viability and normal cell physiology, but with the capacity to favor the return to normal of the actual immunological mechanisms of the host experiencing an acute or chronic inflammatory disease or even some types of cancer.

Eur. J. Med. Chem. 2008, 43, 2404 discloses benzyl-(4,4-diphenyl-but-3-enyl)-amine, 2-[(4,4-diphenyl-but-3-enylamino)-methyl]-phenol, 3-[(4,4-diphenyl-but-3-enylamino)-methyl]-phenol, 5-[(4,4-diphenyl-but-3-enylamino)-methyl]-2-methoxyphenol and 4-[(4,4-diphenyl-but-3-enylamino)-methyl]-2,6-difluoro-phenol as inhibitors of the murine GABA transporters. Eur. J. Med. Chem. 1993, 28, 555, 727 and 783 discloses benzyl-(4,4-diphenyl-but-3-enyl)-amine and vasodilation as a putative therapeutic application of said compound. Org. Lett. 1999, 1, 849 discloses benzyl-(5,5-diphenyl-pent-4-enyl)-ethyl-amine but does not report any biological activity of the compound. US 5,795,756 describes 6-Chloro-9-(4,4-diphenyl-but-3-enyl)-9*H*-purine and 9-(4,4-Diphenyl-but-3-enyl)-9H-purin-6-ylamine, i.e. 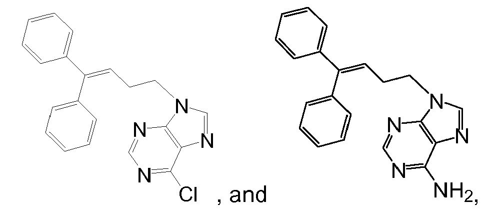 as adenil cyclase inhibitors. Also Also, Falch, E. et al in Drug Dev. Res., 1990, 21, 169-188, describes 5-(4,4-Diphenyl-but-3-enyl)-4,5,6,7-tetrahydro-isoxazolo[4,5-c]pyridin-3-ol 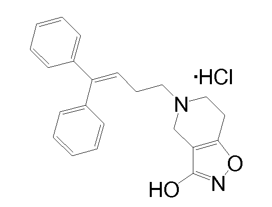 as a GABA uptake inhibitor.

### SUMMARY OF THE INVENTION

The authors of the present invention have surprisingly found that compounds of formula (I) show an interesting activity for immune-modulatory therapy.

Therefore, according to a first aspect, the present invention is directed to a compound of formula (I), or a salt, prodrug or solvate thereof: 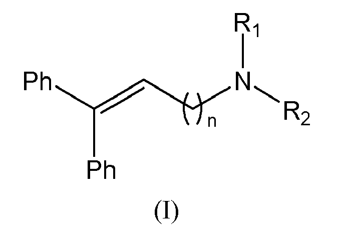 wherein:
Ph is phenyl;
n is 2, 3 or 4;
R₁ is selected from the group consisting of hydrogen and C₁-C₆ alkyl;
R₂ is a radical of formula -[[CH(R₃)]ₘ-R₄], wherein
   m is an integer selected from the group consisting of 1, 0 or 2;
   each R₃, where appropriate, is selected from the group consisting of hydrogen, phenyl and C₁-C₆ alkyl;
   R₄ is selected from the group consisting of an unsubstituted heteroaryl, a substituted heteroaryl and a substituted aryl radical,
   said substituents being selected from the group consisting of C₁-C₆ alkyl, C₇-C₁₁ arylalkyl, phenyl, 5- or 6-membered heteroaryl, F, Cl, Br, I, trifluoromethyl, cyano, -N(Rₐ)(R_{b}), -OR_{c}, -SR_{d} or -C(O)Rₑ; wherein Rₐ, R_{b}, R_{c}, R_{d}, and Rₑ are independently selected from hydrogen, C₁-C₆ alkyl, phenyl and trifluoromethyl;
   or
   if R₁ and/or R₃ are different from hydrogen, then R₄ may also be unsubstituted phenyl;
   or
   R₁ and R₂, together with the nitrogen atom to which they are attached, form a substituted or unsubustited heteroaryl group, wherein said substituents are as defined above;
   with the proviso that:
   2-[(4,4-diphenyl-but-3-enylamino)-methyl]-phenol,
   3-[(4,4-diphenyl-but-3-enylamino)-methyl]-phenol,
   5-[(4,4-diphenyl-but-3-enylamino)-methyl]-2-methoxy-phenol,
   4-[(4,4-diphenyl-but-3-enylamino)-methyl]-2,6-difluoro-phenol,
   benzyl-(5,5-diphenyl-pent-4-enyl)-ethyl-amine,
   6-Chloro-9-(4,4-diphenyl-but-3-enyl)-9H-purine,
   9-(4,4-Diphenyl-but-3-enyl)-9H-purin-6-ylamine, and
   5-(4,4-Diphenyl-but-3-enyl)-4,5,6,7-tetrahydro-isoxazolo[4,5-c]pyridin-3-ol
are not included in formula (I).

According to a further aspect, the present invention is directed to a pharmaceutical composition comprising a compound of formula (I) as defined above, or a pharmaceutically acceptable salt, prodrug or solvate thereof, and at least one pharmaceutically acceptable carrier.

A further aspect of the invention refers to a compound of formula (I) as defined above, or a pharmaceutically acceptable salt, prodrug or solvate thereof, for use as a medicament.

A further aspect of the invention refers to the compounds of formula (I), or a pharmaceutically acceptable salt, prodrug or solvate thereof, 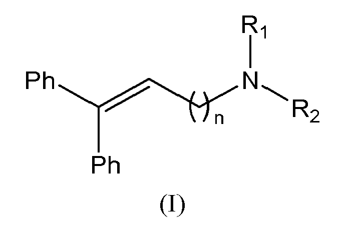 wherein:
Ph is phenyl;
n is 2, 3 or 4;
R₁ is selected from the group consisting of hydrogen and C₁-C₆ alkyl;
R₂ is a radical of formula -[[CH(R₃)]ₘ-R₄], wherein
   m is an integer selected from the group consisting of 1, 0 or 2;
   each R₃, where appropriate, is selected from the group consisting of hydrogen and C₁-C₆ alkyl;
   R₄ is selected from the group consisting of an unsubstituted heteroaryl, a substituted heteroaryl, unsubstitued aryl and a substituted aryl radical,
   said substituents being selected from the group consisting of C₁-C₆ alkyl, C₇-C₁₁ arylalkyl, phenyl, 5- or 6-membered heteroaryl, F, Cl, Br, I, trifluoromethyl, cyano, -N(Rₐ)(R_{b}), -OR_{c}, -SR_{d} or -C(O)Rₑ; wherein Rₐ, R_{b}, R_{c}, R_{d}, and Rₑ are independently selected from hydrogen, C₁-C₆ alkyl, phenyl and trifluoromethyl;
   or
   R₁ and R₂, together with the nitrogen atom to which they are attached, form a substituted or unsubustited heteroaryl group, wherein said substituents are as defined above;
   for use as a medicament for treating inflammatory diseases.

According to a further aspect, the present invention is directed to the use of a compound of formula (I) or a pharmaceutically acceptable salt, prodrug or solvate thereof, in the preparation of a medicament for the treatment of inflammatory diseases.

In a further aspect, the invention is directed to a method of treating inflammatory diseases, said method comprising administering to a patient in need of such a treatment a therapeutically effective amount of at least one compound of formula (I) as described above, or a pharmaceutically acceptable salt, prodrug or solvate thereof.

According to a further aspect, the present invention is directed to a process for the synthesis of the compounds of formula (I), or a pharmaceutically acceptable salt, prodrug or solvate thereof.

### BRIEF DESCRIPTION OF THE FIGURES

Figure 1: chronic lymphocytic leukaemia (CLL) inhibition of compound 3 in a xenograft animal model as compared with a control group treated with vehicle.
Figure 2: multiple myeloma (MM) inhibition of compound 3 in a xenograft animal model as compared with a control group treated with vehicle.
Figure 3: Viavility of cells from patients with chronic lymphocytic leukaemia.

### DETAILED DESCRIPTION OF THE INVENTION

In the context of the present invention, the following terms have the meaning detailed below:
The term "C₁-C₆ alkyl" refers to a linear or branched hydrocarbon chain radical consisting of carbon and hydrogen atoms, containing no insaturation, having between 1 and 6, preferably between 1 and 3 ("C₁₋C₃ alkyl"), carbon atoms and which is attached to the rest of the molecule by a single bond, including for example and in a non-limiting sense, methyl, ethyl, n-propyl, i-propyl, n-butyl, t-butyl, n-pentyl, etc.
The term "aryl" refers to an aromatic group having, unless otherwise provided, between 6 and 18, preferably between 6 and 10, even more preferably 6 or 10 carbon atoms, comprising 1, 2 or 3 aromatic nuclei, bound by means of a carbon-carbon bond or fused, including for example and in a non-limiting sense, phenyl, naphthyl, diphenyl, indenyl, phenanthryl, etc. Preferably "aryl" refers to phenyl.
"Heteroaryl" refers to a stable 3- to 10-membered aromatic ring radical, preferably a 5- or 6-membered aromatic ring, which consists of carbon atoms and from one to five heteroatoms selected from the group consisting of nitrogen, oxygen, and sulphur. For the purposes of this invention, the heteroaryl can be a monocyclic, bicyclic or tricyclic ring system, which can include systems of fused rings, each of them being a stable 3- to 10-membered aromatic ring radical, preferably a 5- or 6-membered aromatic ring, which consists of carbon atoms and from one to five heteroatoms selected from the group consisting of nitrogen, oxygen, and sulphur. The nitrogen, carbon or sulfur atoms in the heteroaryl radical may be optionally oxidized; and the nitrogen atom may be optionally quaternized. Examples of such heteroaryl include, but are not limited to, benzimidazole, benzothiazole, furan, pyrrole, thiophene, pyridine, pyrimidine, isothiazole, imidazole, indole, purine, quinoline, thiadiazole. Preferably, "heteroaryl" refers to furan, thiophene, pyridine and benzimidazole.
"Aralkyl" refers to an aryl group linked to the rest of the molecule by an alkyl group such as benzyl and phenethyl.
The term "halogen" refers to bromo, chloro, iodo or fluoro.

### Compounds of formula (I)

According to a preferred embodiment, "substituted" refers to a radical selected from the group consisting of C₁-C₆ alkyl, C₇-C₁₁ arylalkyl, phenyl, 5- or 6-membered heteroaryl, F, Br, I, trifluoromethyl, cyano, -N(Rₐ)(R_{b}), -OR_{c}, -SR_{d} or -C(O)Rₑ; wherein Rₐ, R_{b}, R_{d}, and Rₑ are independently selected from hydrogen, C₁-C₆ alkyl, phenyl and trifluoromethyl; and R_{c} is selected from the group consisting of C₁-C₆ alkyl, phenyl and trifluoromethyl, provided that -N(Rₐ)(R_{b}) is not NH₂. Substituted radicals, e.g. aryl or heteroaryl, may be so in any of their free positions. In an embodiment of the invention, substituted radicals are substituted in 1, 2, 3 or 4 of their positions, preferably in 1 or 2.

According to an embodiment of the invention the substituents are selected from the group consisting of methyl, isopropyl, phenylmethyl, phenyl, thiophene, pyridine, F, Br, I, trifluoromethyl, cyano, amino, methoxi, trifluoromethoxi, and thiometoxi.

An embodiment of the invention is directed to a compound of formula (IA), or a salt, prodrug or solvate thereof, 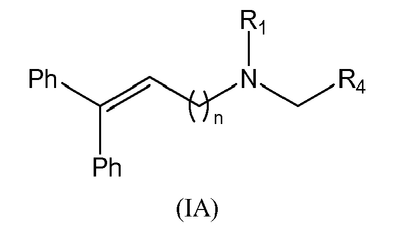 wherein Ph, n, R₁ and R₄ are as defined above.

According to an embodiment of the invention, R₄ is an heteroaryl selected from the group consisting of thienyl, furyl, pyridil, 1*H*-benzimidazol, 9*H*-Purine, 1*H-*Imidazole, and 1*H*-Pyrazolo[3,4-*d*]pyrimidine, wherein each group may be substituted as defined above.

A further embodiment of the invention is a compound of formula (I) or (IA) or the salts, prodrugs or solvates thereof, wherein R₄ is a group of formula (V) or (VI) 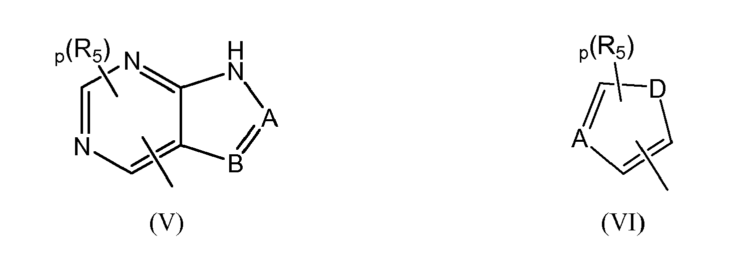 wherein,
A and B are independently selected from -CH- and -N-;
D is independently selected from the group consisting of -O-, -S- and -NH-;
p is an integer selected from the group consisting of 0, 1, 2 or 3;
each R₅ is selected from the group consisting of C₁-C₃ alkyl, phenyl, phenylmethyl, 5-or 6-membered heteroaryl, F, Cl, Br, I, trifluoromethyl, -N(Rₐ)(R_{b}), -SR_{d} or -C(O)Rₑ; wherein Rₐ, R_{b}, R_{d}, and Rₑ are independently selected from hydrogen, C₁-C₃ alkyl, phenyl and trifluoromethyl, provided that in a compound of formula (V) -N(Rₐ)(R_{b}) is not -NH₂;
said group of formula (V) or (VI) being attached to the rest of the molecule through any of the free positions.

According to an embodiment of the invention, each R₅, if any, is independently selected from the group consisting of methyl, isopropyl, phenylmethyl, phenyl, thiophene, pyridine, F, Cl, Br, I, trifluoromethyl, cyano, amino, methoxi, trifluoromethoxi, and thiometoxi. According to a further embodiment, p is 1 or 2.

According to an embodiment of the invention R₄ is a group of formula (VII) 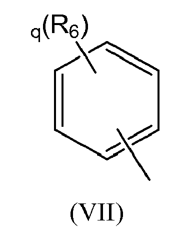 wherein R₆ is selected from the group consisting of -OCF₃, OC₁-C₃alkyl, F, Cl, Br, I and -CN; and q is an integer selected from the group consisting of 0, 1, 2 or 3, and said group of formula (VII) is attached to the rest of the molecule through any of the free positions.

According to an embodiment of the invention, R₁ and R₂, together with the nitrogen atom to which they are attached, form a radical selected from the group consisting of 1*H*-benzimidazol, 9*H*-Purine, 1*H*-Imidazole, and 1*H*-Pyrazolo[3,4-*d*]pyrimidine.

According to an embodiment of the invention R₁ is hydrogen or methyl.

An embodiment of the invention is also directed to a compound selected from the group consisting of: 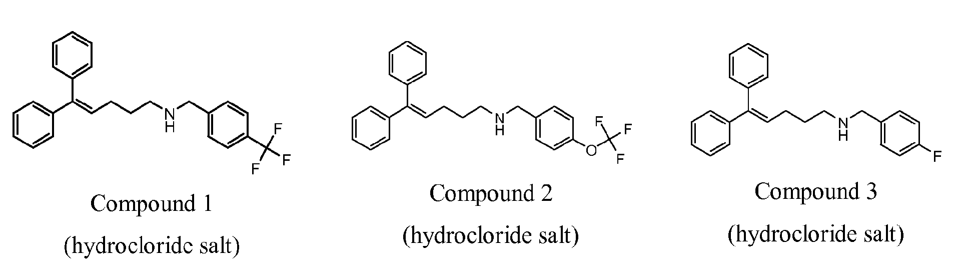 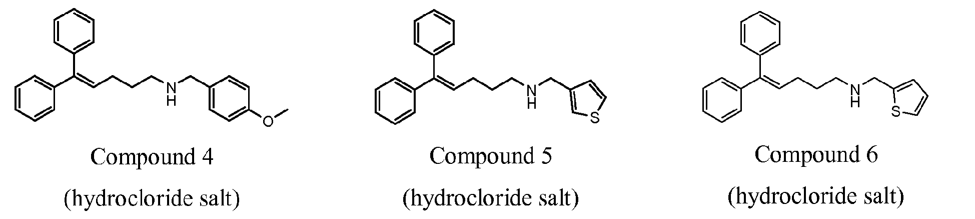 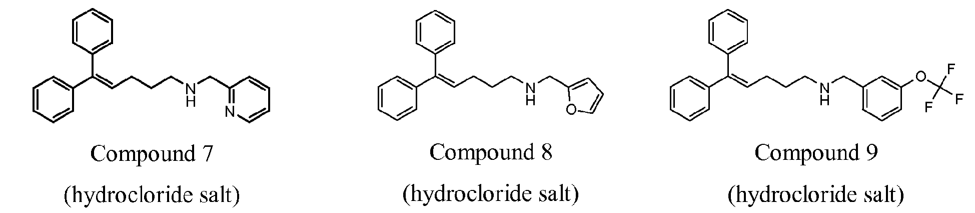 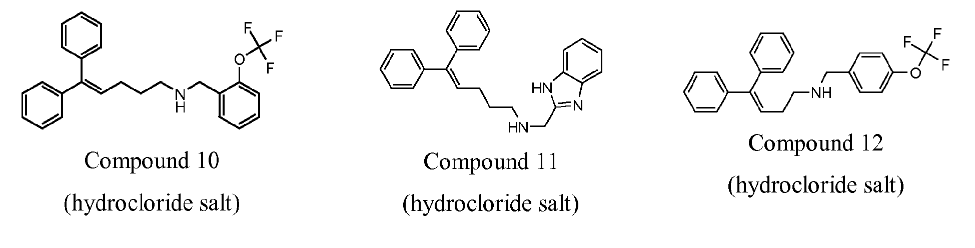 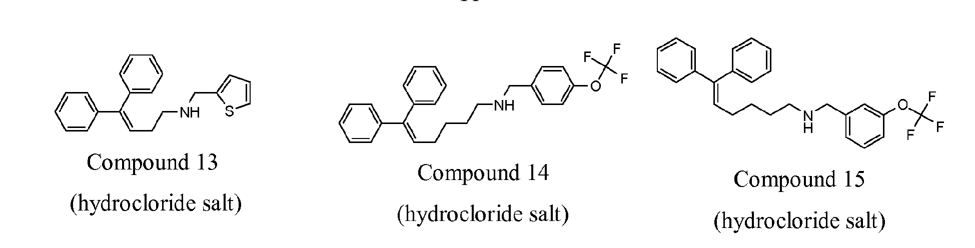 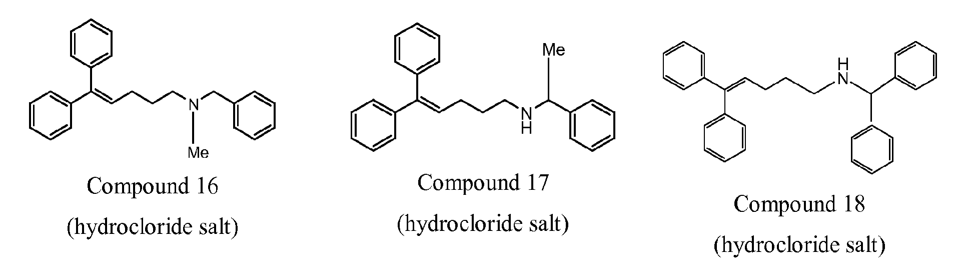 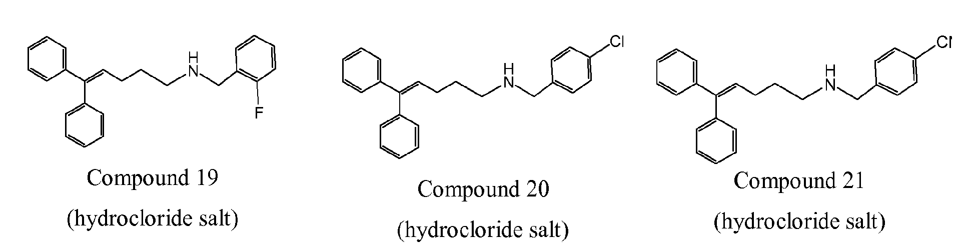 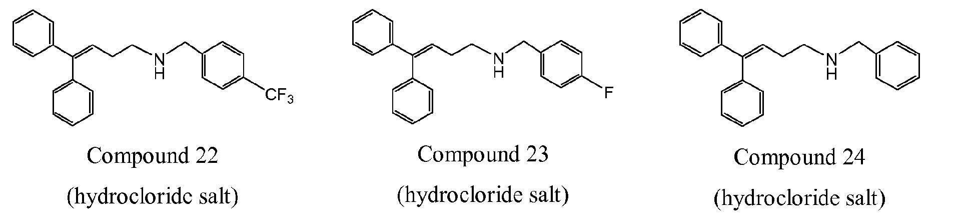 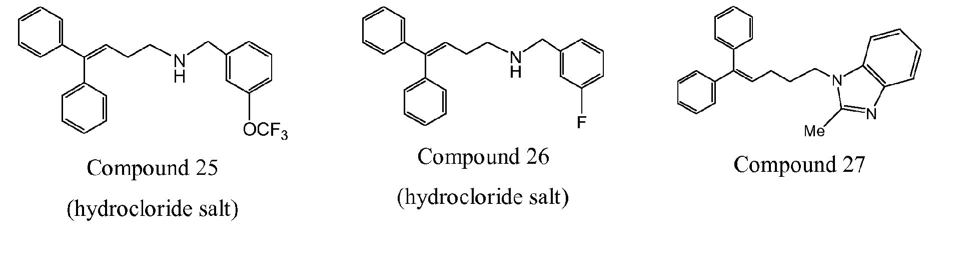 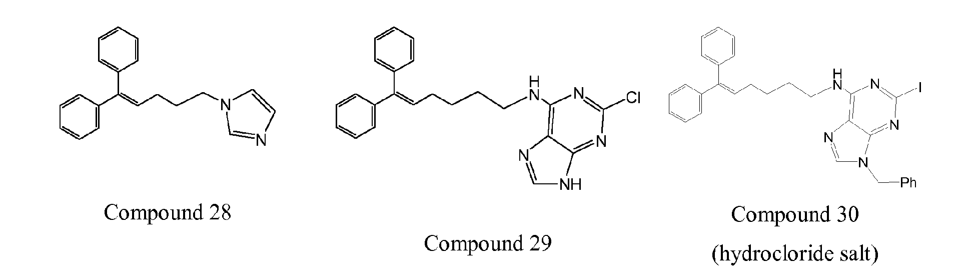 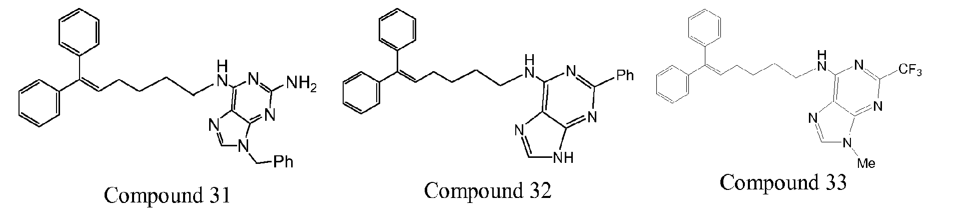 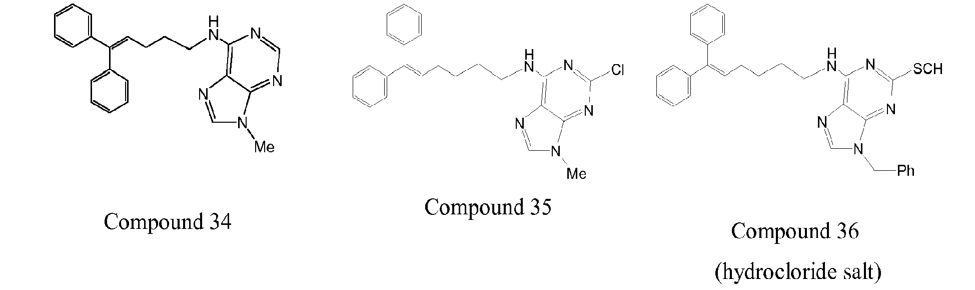 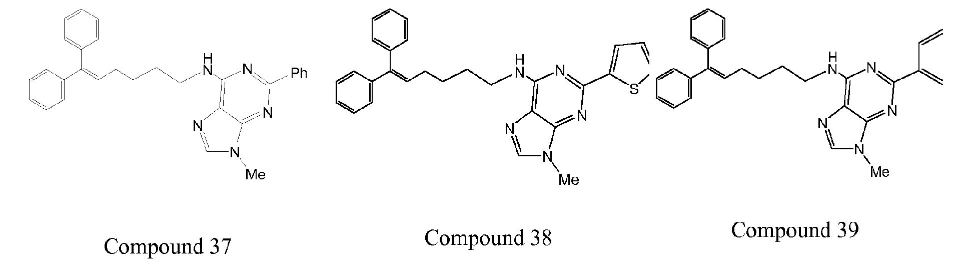 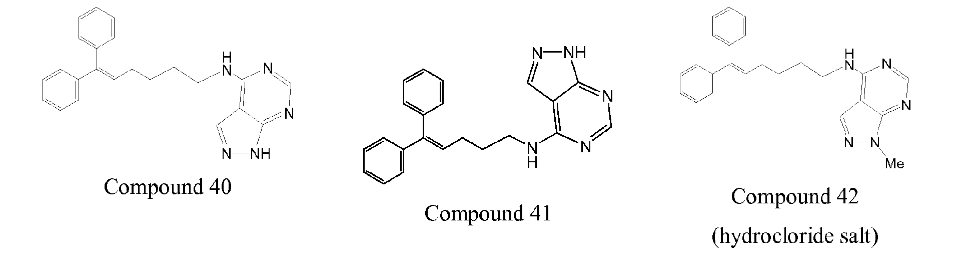 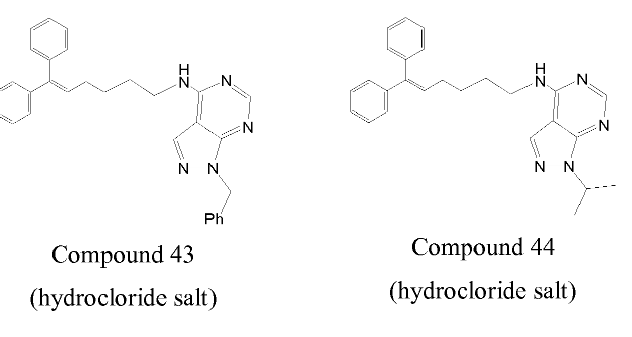 or a salt, prodrug and/or solvate thereof.

The compounds of formula (I) may be in the form of salts, preferably pharmaceutically acceptable salts, in the form of solvates or in the form of prodrugs. The term "pharmaceutically acceptable salts, solvates or prodrugs thereof" relates to salts, solvates or prodrugs which, when administered to the recipient, can provide (directly or indirectly) a compound such as the one described herein. Nevertheless, it will be observed that pharmaceutically unacceptable salts are also within the scope of the invention because they can be useful for preparing pharmaceutically acceptable salts. Salts, prodrugs and derivatives can be prepared by means of methods known in the state of the art. "Pharmaceutically acceptable" preferably relates to molecular entities and compositions which are physiologically tolerable and do not typically cause an allergic reaction or a similar unfavorable reaction, such as gastric disorders, dizziness and the like, when administered to a human or animal. The term "pharmaceutically acceptable" means that it is approved by a regulatory agency of a federal or state government or is included in the US pharmacopoeia or another generally recognized pharmacopoeia for use in animals, and more particularly in humans.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate. Preferably, the solvates are pharmaceutically acceptable solvates.

The preparation of salts and solvates can be carried out by methods known in the art. For instance, pharmaceutically acceptable salts of compounds provided herein are synthesized from the parent compound, which contains one or more basic moieties, by conventional chemical methods. Generally, such salts are, for example, prepared by reacting the free base forms of these compounds with the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. In a preferred embodiment, the salt is the hydrochloride or fumarate salt.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

Any compound that is a prodrug of a compound of formula (I) is within the scope of the invention. The term "prodrug" is used in its broadest sense and encompasses those derivatives that are converted in vivo to the compounds of the invention. Examples of well known methods of producing a prodrug of a given acting compound are known to those skilled in the art and can be found e.g. in Krogsgaard-Larsen et al., Textbook of Drug design and Discovery, Taylor & Francis (April 2002). Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or a nitrogen by ¹⁵N-enriched nitrogen are within the scope of this invention.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

### Uses of compounds of formula (I)

According to a preferred embodiment, the inflammatory disease is selected from Inflammatory Bowel Disease (IBD), Rheumatoid Arthritis (RA), benign prostatic hyperplasia, Barrett's disease, asthma, skeletal muscle and tendon repair, ulcerative colitis, leishmaniasis and autoimmune diseases, preferably Crohn's disease. According to a further embodiment, the disease is cancer, for example, selected from the group consisting of metastasis, breast cancer, esophageal cancer, colon cancer, colon carcinomas, stomach cancer, Leukemias, Melanoma, carcinomas of the uterus, non-small cell lung cancer, small cell lung cancer, ovarian cancer, ovarian carcinomas, prostate cancer, renal cancer, liver cancer, carcinomas of the pancreas, kidney cancer, bladder cancer, prostate cancer, testicular cancer, bone cancer, skin cancer, sarcoma, Kaposi sarcomas, brain tumours, myosarcomas, neuroblastomas, limphomas and multiple myeloma.

The term "treatment" or "to treat" or "treating" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

### Pharmaceutical compositions

According to a further aspect, the present invention is directed to a pharmaceutical composition comprising a compound of formula (I) as defined above, or a pharmaceutically acceptable salt, prodrug or solvate thereof, and at least one pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition. Preferably, the carriers of the invention are approved by a regulatory agency of the Federal or a state government or listed in the U.S. Pharmacopeia or other generally recognized pharmacopeia for use in animals, and more particularly in humans.

The carriers and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition.

Examples of pharmaceutical compositions include any solid (tablets, pills, capsules, granules etc.) or liquid (solutions, suspensions or emulsions) compositions for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 1000 mg/kg/day.

These pharmaceutical compositions of the invention can be used in combination with other active ingredients, such as further compounds for use in the treatment of inflammatory diseases. Said compositions can be used, as preparations of each component (a compound according to formula (I) or a pharmaceutically acceptable salt, prodrug or solvate thereof and a further active ingredient), for their administration in a simultaneous, separately or sequential way.

### Synthesis of compounds of formula (I)

The compounds of the present invention can be synthesized in a multi-step sequence by available synthetic procedures. For example, they can be prepared by the process summarized in the general scheme 1 shown below:

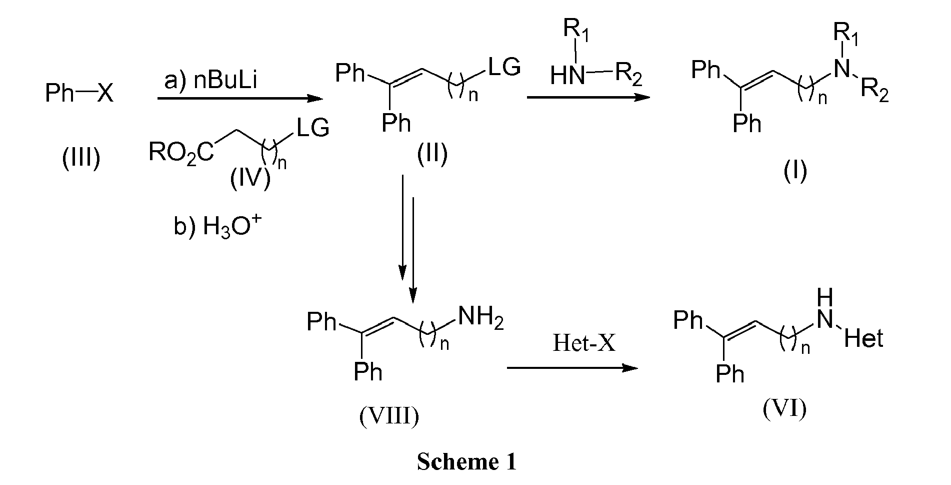

In a particular embodiment, the compounds of formula (I) can be prepared by substitution reaction of the derivative (II) with an amine carrying the R₁ and R₂ radicals in the presence of a base and an organic solvent. According to a preferred embodiment, diisopropylethylamine or potassium carbonate are used as base. According to another preferred embodiment, the organic solvent is acetonitrile or ethanol. The leaving group (LG) present in compounds (II) is preferably selected from halide and sulfonate. More preferably, it is bromide.

Additionally, when the amine is a primary amine (R₁ = H), the resulting compound (I) can be further alkylated by treatment with an alkyl halide (R₁-X; wherein R₁ is C₁-C₆alkyl or C₇-C₁₅ aralkyl) in the presence of a base.

In a particular embodiment, the compounds of formula (II) can be prepared by addition of the phenyl- lithium halide to an ester of formula (IV) and further dehydration reaction. According to a preferred embodiment, the organolithium compound is prepared by addition of n-butillithium to the phenyl halide. According to another preferred embodiment, the dehydration step is performed in the presence of an acid. In a preferred embodiment, the acid is H₂SO₄. Preferably the synthesis of compounds (II) is performed in the presence of an ethereal solvent. More preferably, it is selected from diethyl ether and tetrahydrofuran.

In a particular embodiment, the compounds of formula (VI), i.e. a compound of formula (I) wherein R1 is hydrogen, R₂ is -[[CH(R₃)]ₘ-R₄]-, m being 0 and R₄ being a substituted or unsubstituted heteroaryl as defined herein, can be can be prepared by reaction of an amine of formula (VIII) with the corresponding substituted or unsubstituted halogenated heteroaryl derivative (Het-X; X being F, Br, Cl or I) in the presence of a base and an organic solvent. According to a preferred embodiment, diisopropylethylamine or potassium carbonate are used as base. According to another preferred embodiment, the organic solvent is ethanol. Further conditions for the alkylation of amines are known to the skilled person.

In a particular embodiment, the compounds of formula (VIII) can be prepared by reaction of a compound (III) with sodium azide in DMF and subsequent reduction of this azides with triphenylphosphine in THF.

The present invention is additionally explained below by means of examples. This explanation must by no means be interpreted as a limitation of the scope of the invention as it is defined in the claims.

### EXAMPLES

Compounds of formula (I) according to the present invention were prepared following the general preparation strategy detailed below. The detailed preparation of some of the compounds is described hereinafter. All the reactants used are commercially available.

### Example 1: Synthesis of (5,5-diphenyl-pent-4-enyl)-(4-fluoro-benzyl)-amine hydrocloride (Compound 3)

### Step 1. Synthesis of 5-bromo-1,1-bisphenylpentene

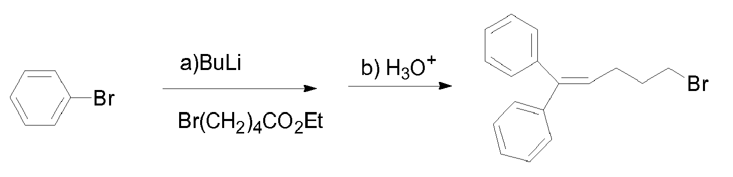

A solution of bromobenzene (20.86 mL, 0.225 mol) in anhydrous Et₂O (60 mL) was added, under argon atmosphere, to a mixture of 2.5M *n*-butyllithium (100 mL, 0.250 mol) in anhydrous Et₂O (100 mL) while keeping the temperature at 5-10 °C. Stirring was continued at 10 °C for another 15 min before the mixture was cooled down to -70 °C. A solution of ethyl 5-bromovalerate (14.54 mL, 0.090 mol) in anhydrous Et₂O (60 mL) was added while keeping the temperature below -65 °C. When the addition was completed the mixture was stirred at -70 °C for 2.5 h. Cold water (75 mL) and cold aqueous 1 N HCl (35 mL) were added successively while the temperature was kept bellow 0 °C. The reaction mixture was stirred for 15 min to allow the temperature to rise at >0 °C, and the phases were separated. The aqueous phase was extracted with Et₂O (75 mL), and the combined organic phases were washed with water (60 mL) and brine (60 mL). The solution was dried over anhydrous sodium sulphate and the solvent was evaporated under vacuum to give 46.5 g of a solid, which was dissolved in 2-propanol (225 mL). A 20% aqueous H₂SO₄ solution (25 mL) was added, and the mixture was stirred under reflux for 1 h. The solvents were evaporated under vacuum to give a residue which was partitioned between CH₂Cl₂ (400 mL) and a saturated NaHCO₃ solution (75 mL). The phases were separated, and the aqueous phase was extracted further with CH₂Cl₂ (75 mL). The combined organic phases were washed with water (75 mL), brine (75 mL), and dried over anhydrous sodium sulphate. The solvent was evaporated under vacuum to give 5-bromo-1-1-bisphenylpentene (20.0 g, 73.7%) as a yellow oil. ¹H NMR (CDCl₃), δ(ppm): 7.2-7.4 (m, 10 H); 6.2 (t, 1H); 3.3 (t, 2H); 2.3 (q, 2H); 1.9 (q, 2H).

### Further compounds:

### Step 2. Synthesis of (5,5-diphenyl-pent-4-enyl)-(4-fluoro-benzyl)-amine

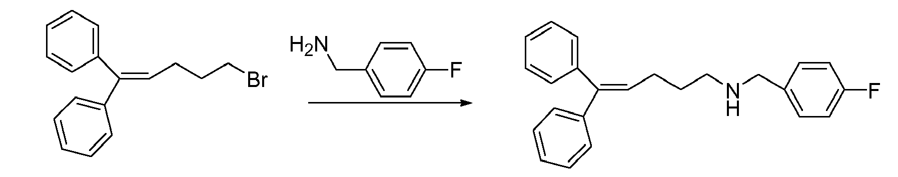

4-Fluoro-benzylamine (0.62 g, 5 mmol) and K₂CO₃ (0.7 g, 5 mmol) were added to a solution of 5-bromo-1-1-bisphenylpentene (0.75 g, 2.5 mmol) in 25 mL of acetonitrile. The reaction was heated to reflux under stirring for 16 hours. The solvent was removed under reduced pressure, water was added and the mixture was extracted with CH₂Cl₂ (3 x 50 mL). The organic layers were dried over anhydrous sodium sulphate and the solvent was evaporated under vacuum to give an oil which was purified by flash chromatography (AcOEt/Heptane/TEA 45:55:1) to give 0.58 g (67.1% yield) of (5,5-diphenyl-pent-4-enyl)-(4-fluoro-benzyl)-amine as a yellow oil. ¹H NMR (CDCl₃), δ(ppm): 7.2-7.5 (m, 12H); 7.0-7.1 (m, 2H); 6.2 (t, 1H); 3.8 (s, 2H); 2.7 (t, 2H); 2.3 (c, 2H); 1.7 (q, 2H). ¹³C NMR (CDCl₃), δ(ppm): 163.2; 143.4; 141.9; 139.5; 132.5; 132.3; 129.6; 128.3; 128.1; 127.1; 126.6; 125.7; 116.3; 115.9; 49.6; 45.3; 26.7; 25.8.

### Step 3. Synthesis of (5,5-diphenyl-pent-4-enyl)-(4-fluoro-benzyl)-amine hydrocloride (Compound 3)

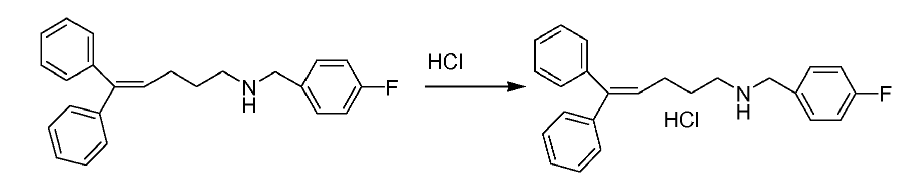

A solution of HCl 1M in anhydrous Et₂O (3 mL, 3 mmol) was added within 10 min. to a solution of (5,5-Diphenyl-pent-4-enyl)-(4-fluoro-benzyl)-amine (1 g, 3 mmol) in 100 mL of anhydrous Et₂O. The reaction was stirred for 30 min. The solid was filtered, transferred to a crystallising dish and dried at 50 °C in a vacuum oven (0.9 g, 84.7% yield). MP= 128.0-130.1 °C. ¹H NMR (CDCl₃), δ(ppm): 9.9 (bs, 2H); 7.6-7.5 (m, 2H); 7.3-7.0 (m, 12H); 5.9 (t, 1H); 4.0 (bs, 2H); 2.7 (t, 2H); 2.2-1.9 (m, 4H). ¹³C NMR (CDCl₃), δ(ppm): 163.2; 143.4; 141.9; 139.5; 132.5; 132.3; 129.6; 128.3; 128.1; 127.1; 126.6; 125.7; 116.3; 115.9; 49.6; 45.3; 26.7; 25.8.

The following compounds were obtained following the general synthetic process described above for compounds of formula (I), concretely by reaction of 5-bromo-1-1-bisphenylpentene with corresponding arylbenzylamines:
**Example 2: (5,5-diphenyl-pent-4-enyl)-(4-trifluoromethoxybenzyl) amine hydrochloride (Compound 2)** ¹H-NMR (CDCl₃), δ (ppm): 7.2 (m, 14H); 6.1 (t, 1H); 3.8 (s, 2H); 2.7 (t, 2H); 2.2 (q, 2H); 1.7 (m, 2H).
**Example 3: (5,5-diphenyl-pent-4-enyl)-(4-trifluoromethylbenzyl)amine hydrochloride (Compound 1).** m.p.: 121.4-122.7 °C.
   ¹H-NMR (CDCl₃), δ (ppm): 10.1 (s, 2H,); 7.7-7.6 (m, 4H); 7.0-6.8 (m, 10H); 5.9 (t, 2H,); 4.0 (s, 2H); 2.6 (bs 2H); 2.1-1.9 (m, 4H).
**Example 4: (5,5-diphenyl-pent-4-enyl)-(4-methoxybenzyl)amine hydrochloride (Compound 4)** m.p.: 69.2-73.8 °C.
   ¹H-NMR (CDCl₃), δ (ppm): 9.7 (bs , 2H); 7.4 (d,2H); 7.3-7.0 (m, 10H); 6.8 (d, 2H); 5.9 (t, 1H); 3.9 (bs 2H); 3.7 (s, 3H); 2.6 (bs, 2H,); 2.1-1.9 (m, 4H).
**Example 5: (5,5-diphenyl-pent-4-enyl)-thiophen-3-ylmethylamine hydrochloride (Compound 5).** m.p.: 108.0-109.7 °C. ¹H-NMR (CDCl₃) δ (ppm): 9.8 (bs, 2H); 7.5-7.4 (m, 1H); 7.3-7.0 (m, 12H); 5.9 (t, 1H); 4.0 (bs , 2H); 2.7 (bs, 2H); 2.1-1.9 (m, 4H).
**Example 6: (5,5-diphenyl-pent-4-enyl)-thiophen-2-ylmethyl-amine hydrochloride (Compound 6).** m.p.: 161.0-161.6 °C. ¹H-NMR (CDCl₃), δ (ppm): 7.4-7.1 (m, 11H); 6.9 (m, 2H;); 6.1 (t, 1H); 4.0 (s, 2H); 2.7 (t, 2H); 2.2 (q, 2H); 1.6 (m, 2H).
**Example 7: (5,5-diphenyl-pent-4-enyl)pyridin-2-ylmethylamine hydrochloride (Compound 7).** m.p.: 125.0-126.9 °C. ¹H-NMR (CDCl₃), δ (ppm): 8.5 (m, 1H); 7.8 (m, 2H); 7.4-7.0 (m, 11H); 5.9 (t,1H); 4.4 (s, 2H); 2.9 (t, 2H); 2.2-2.0 (m, 4H).
**Example 8: (5,5-diphenyl-pent-4-enyl)furan-2-ylmethylamine hydrochloride (Compound 8).** m.p.: 139.0-141.3 °C. ¹H-NMR (DMSO-d₆), δ (ppm): 9.5 (bs, 2H); 7.7 (m, 1H); 7.5-7.0 (m, 10H); 6.6(m, 1H); 6.5 (m, 1H); 6.0 (t, 1H); 4.1 (bs, 2H); 2.8 (bs, 2H); ); 2.1-2.0 (m, 2H);1.8-1.7 (m, 2H).
**Example 9: (5,5-diphenyl-pent-4-enyl)-(3-trifluoromethoxy-benzyl)amine hydrochloride (Compound 9).** m.p.: 102.4-104.0 °C. ¹H-NMR (CDCl₃), δ (ppm): 10.0 (bs 2H); 7.6-7.0 (m, 14H); 5.9 (t, 1H); 4.0 (s, 2H); 2.7 (bs, 2H); 2.1-1.9 (m, 4H).
**Example 10: (5,5-diphenyl-pent-4-enyl)-(2-trifluoromethoxybenzyl)amine hydrochloride (Compound 10).** m.p.: 109.0-110.7 °C. ¹H-NMR (CDCl₃), δ (ppm): 10.0 (bs, 2H), 8.0 (m, 1H); 7.4-7.0 (m, 13H); 5.9 (t, 1H); 4.2 (s, 2H); 2.7 (bs, 2H); 2.1-2.0 (m, 4H).
**Example 11: (1H-benzoimidazol-2-ylmethyl)-(5,5-diphenyl-pent-4-enyl)amine hydrochloride (Compound 11).** m.p.: 251.0-253.8 °C. ¹H-NMR (DMSO-d₆), δ (ppm): 10.1 (bs, 2H); 7.8-7.1 (m, 14H); 6.2 (bs 2H,); 6.1 (t,1H); 4.6 (s, 2H); 3.0 (bs, 2H); 2.1-2.0 (m, 2H);1.8-1.0 (m, 2H).
**Example 12: (4,4-diphenyl-but-3-enyl)-(4-trifluoromethoxybenzyl)amine hydrochloride (Compound 12).** m.p.: 126-128.°C. ¹H-NMR (CDCl₃), δ (ppm): 10.1 (s, 1H); 6.1 (t, 1H); 7.5 (d, 2H); 7.3-7.0 (m, 12H); 3.9 (s, 2H); 2.8 (m, 2H); 2.6 (m, 2H).
**Example 13: (4.4-diphenyl-but-3-enyl)-thiophen-2-ylmethyl-amine hydrochloride (Compound 13).** m.p.: 163-165.5 °C. ¹H-NMR (CDCl₃), δ(ppm): 2.3 (q, 2H); 2.8 (t, 2H); 4.0 (s, 2H); 6.1 (t, 1H); 6.9 (m, 2H); 7.0 (m, 8H); 7.4 (m, 3H).
**Example 14: (6,6-diphenyl-hex-5-enyl)-(4-trifluoromethoxybenzyl)amine hydrochloride (Compound 14).** m.p.: 109-110.5 °C. ¹H-NMR (CDCl₃), δ(ppm): 10.0 (m, 2H); 7.6 (d, 2H); 7.4-7.0 (m, 14H); 6.0 (t, 1H); 3.9 (s, 2H); 2.7 (s, 2H); 2.1 (q, 2H); 1.8 (m, 2H); 1.5 (m, 2H).
**Example 15: (6,6-diphenyl-hex-5-enyl)-(3-trifluoromethoxybenzyl)amine hydrochloride (Compound 15).** m.p.: 96.6-98.5 °C. ¹H-NMR (CDCl₃), δ(ppm): 7.4-7.0 (m, 13H); 6.1 (t,1H); 3.8 (s, 2H); 2.6 (t, 2H), 2.2 (q, 2H); 1.6 (m, 4H).
**Example 16: Benzyl-(5,5-diphenyl-pent-4-enyl)-methyl-amine hydrochloride (Compound 16).** ¹H-NMR (CDCl₃), δ(ppm): 12.5 (s, 1H); 7.0-7.6 (m, 15H); 5.9 (t, 1H); 4.1 (s, 2H); 2.9-2.6 (m, 2H); 2.6 (s, 3H); 2.2-1.8 (m, 4H).
**Example 17: (5,5-Diphenyl-pent-4-enyl)-(1-phenyl-ethyl)-amine hydrochloride (Compound 17).** ¹H-NMR (CDCl₃), δ (ppm): 10.1 (bs 1H); 9.8 (bs, 1H); 7.6-7.0 (m, 15 H); 5.9 (t, 1H); 4.16 (t, 1H); 2.6 (bs, 2H); 2.0 (m, 4H); 1.9 (d, 3H).
**Example 18: Benzhydryl-(5,5-diphenyl-pent-4-enyl)-amine hydrochloride (Compound 18).** ¹H-NMR (CDCl₃), δ (ppm): 10.3 (bs, 2H); 7.6-7.0 (m, 20H); 5.8 (t, 1H); 5.1 (bs,1H); 2.5 (bs 2H); 1.9-1.7 (m, 4H).
**Example 19: (5,5-Diphenyl-pent-4-enyl)-(2-fluorobenzyl)amine hydrochloride (Compound 19).**¹H- NMR (CDCl₃) δ (ppm): 10.0 (s, 2H); 7.4-6.9 (m, 15H); 6.0 (t, 1H); 4.0 (s, 2H); 2.7 (m, 2H); 2.2-2.0 (m, 4H).
**Example 20: (4-Chlorobenzyl)-(5,5-diphenyl-pent-4-enyl)amine hydrochloride. (Compound 20).** ¹H- NMR (CDCl₃), δ (ppm): 1.7 (m, 2H); 2.2 (m, 2H); 2.7 (m, 2H); 4.0 (s, 2H); 6.1 (t, 1H); 7.5-7.0 (m, 14H); 10.0 (bs, 2H).
**Example 21: 4-[(5,5-Diphenyl-pent-4-enylamino)-methyl]benzonitrile hydrochloride** ¹H-NMR (CDCl₃), δ (ppm): 10.2 (s, 2H); 7.8-7.6 (m,4H); 7.4-7.0 (m, 10H); 5.9 (t, 1H); 4.1 (s, 2H); 2.7 (m, 2H); 2.2 (m, 2H); 2.0 (m,2H).
**Example 22: (4,4-Diphenyl-but-3-enyl)-(4-trifluoromethylbenzyl)amine hydrochloride.** ¹H-NMR (CDCl₃) δ (ppm): 11.0 (bs, 2H); 7.6 (m, 4H); 7.4-7.2 (m, 8H); 7.1(m, 2H); 6.1 (t, 1H); 4.0 (s, 2H); 2.9 (t, 2H); 2.6 (m, 2H).
**Example 23: (4,4-Diphenyl-but-3-enyl)-(4-fluor-benzyl-amine hydrochloride.** ¹H-NMR (CDCl₃), δ (ppm): 10.0 (s, 2H); 7.6-7.4 (m, 10H); 7.0-6.8 (m, 4H); 6.1 (t, 1H); 3.9 (m, 2H); 2.8 (m, 2H); 2.5 (m,2H).
**Example 24: Benzyl-(4,4-diphenyl-but-3-enyl)amine hydrochloride.** ¹H-NMR (CDCl₃), δ (ppm): 10.0 (bs, 2H); 7.5-7.2 (m, 13H); 7.0 (m, 2H); 6.1 (t, 1H); 3.9 (s 2H); 2.9 (m,2H); 2.6 (m, 2H).
**Example 25: (4,4-Diphenyl-but-3-enyl)-(3-trifluoromethoxylbenzyl)amine hydrochloride.** ¹H-NMR (CDCl₃, δ (ppm): 10.2 (bs, 2H); 7.8 (d, 1H); 7.6 (m, 2H); 7.5 (m, 1H); 7.7-7.4 (m, 10H); 6.1 (t, 1H); 4.0 (s, 2H); 2.9 (m, 2H); 2.6 (m, 2H).
Example 26: (4,4-Diphenyl-but-3-enyl)-(3-fluorobenzyl)amine hydrochloride. ¹H-NMR (CDCl₃), δ (ppm): 10.0 (bs, 2H); 7.4-7.7 (m, 14H); 6,1 (t, 1H); 4.0 (s, 2H); 2.9 (m, 2H); 2.6 (m, 2H).
Example 27: Synthesis of (1-(5,5-Diphenyl-pent-4-enyl)-2-methyl-1*H-*benzoimidazole (Compound 27). 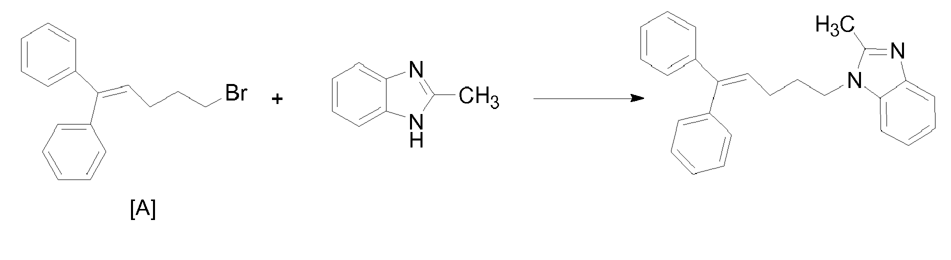 Sodium hydride (0.24 g, 60% in oil dispersion, 6.0 mmol) was slowly added portionwise onto a precooled solution (ice/water cooling bath) of 2-methylbenzimidazole (0.66 g, 5.0 mmol) in 20 mL of anhydrous DMF. After 2 h, 5-bromo-1,1-bisphenylpentene (1.5 g, 5.0 mmol) was added portionwise, and the resulting mixture was stirred for 20h. The reaction was quenched by addition of methanol (3 mL), until cessation of gas evolution. It was diluted with water (120 mL) and extracted with ethyl acetate (3 x 25 mL). The organic extract was washed with brine (1 x 20 mL), dried over anhydrous sodium sulfate and the solvent eliminated under low pressure, to obtain a residue (1.9 g), which was purified by flash chromatography (DCM/MeOH 98:2) to give 1.5 g (4.2 mmol, 60% yield) of a yellow solid. ¹H-NMR (CDCl₃), δ (ppm): 7.7 (m, 1H); 7.4-7.0 (m, 13H); 6.0 (t, 1H); 4.0 (t, 2H); 2.5 (s, 3H); 2.3 (q, 2H); 1.9 (m, 2H). The following compounds were prepared in an analogous way:
**Example 28: 1-(5,5-Diphenyl-pent-4-enyl)-1*H*-imidazole (compound 28).** ¹H-NMR (CDCl₃), δ (ppm): 7.5-7.2 (m, 11H); 7.0 (s, 1H); 6.8 (s, 1H); 6.1 (t, 1H); 3.9 (t, 2H); 2.2 (q, 2H); 1.9 (m, 2H).
**Example 29: Synthesis of (2-Chloro-9*H*-purin-6-yl)-(6,6-diphenyl-hex-5-enyl)-amine (Compound 29)**
   Step 1 . Synthesis of 6-azide-1,1-diphenyl-1-hexene Intermediate [D] 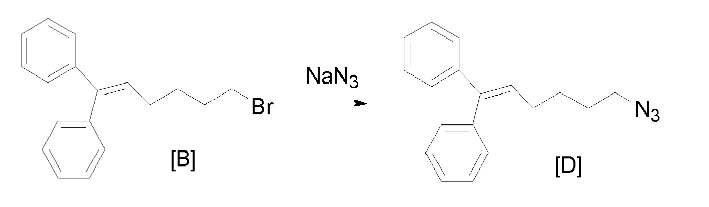 A mixture of 6-bromo-1,1-diphenyl-1-hexene (50.0 g, 158 mmol) and sodium azide (31.0 g, 476 mmol) DMF (250 mL) was stirred at ambient temperature for 16h. The reaction mixture was diluted with ethyl acetate (400 mL) and washed with a saturated aqueous solution of NH₄Cl (3 x 100 mL). The organic extract was washed with brine (1x 60 mL), dried over anhydrous sodium sulfate and the solvent was eliminated at low pressure, to obtain 41g (148 mmol, 96.68%) of a colorless oil, identified as 6-azide-1,1-diphenyl-1-hexene . ¹H-NMR (CDCl₃), δ (ppm): 7.4-7.1 (m, 10H); 6.1 (t, 1H); 3.2 (t, 2H); 2.1 (m, 2H); 1.5 (m, 4H).
   The following intermediate compound was prepared by reaction of intermediate [A] (see example 27) with sodium azide in an analogous way:
   Intermediate [E] ***5-azide-1,1-diphenyl-1-pentene*** ¹H-NMR (CDCl₃), δ (ppm): 7.4-7.0 (m, 10H); 6.1 (t, 1H); 3.3 (t, 2H); 2.1 (m, 2H); 1.6 (m, 2H).
   Step 2 . Synthesis of 6,6-Diphenyl-hex-5-enylamine. Intermediate [F] 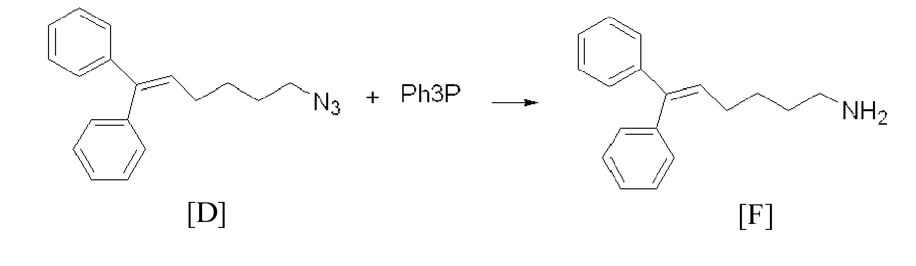 A mixture of of 6-azide-1,1-diphenyl-1-hexene (2.7 g, 9.7 mmol) and triphenylphosphine (3.1 g, 12 mmol) in THF (50 mL) and water (2.5 mL), was stirred at ambient temperature for 20h. The solvent was removed under reduced pressure to give an oil, which was purified by flash chromatography (DCM/MeOH/Et₃N 95:5:5) to yield of 6,6-diphenyl-hex-5-enylamine as a yellow oil (2.4 g, 98% yield) ¹H-NMR (CDCl₃), δ (ppm): 1.4 (m, 4H); 7.3 (m, 10H); 6.1 (t, 1H); 2.6 (m, 2H); 2.2 (q, 2H); 1.6 (s, 2H).
   The following intermediate compound was prepared in an analogous way:
   Intermediate [G] **5,5-Diphenyl-pent-4-enylamine** ¹H-NMR (CDCl₃), δ (ppm): 7.4-7.2 (m, 10H); 6.1 (t, 1H); 2.7 (t, 2H); 2.2 (q, 2H); 1.9 (s, 2H); 1.6 (m,2H).
   Step 3. (2-Chloro-9*H*-purin-6-yl)-(6,6-diphenyl-hex-5-enyl)-amine 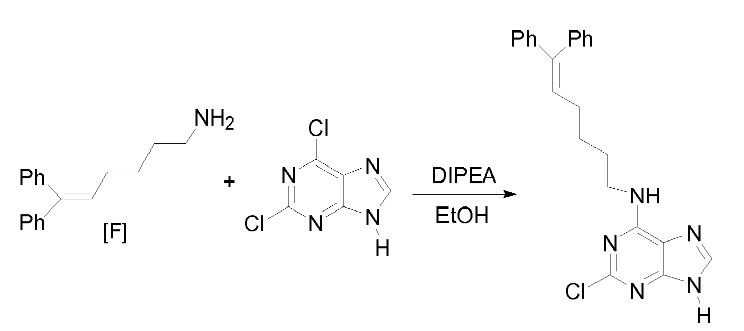 To a solution of 6,6-diphenyl-hex-5-enylamine (25.6 g, 103 mmol) in ethanol (500 mL), purine (17.0 g, 90 mmol) and DIPEA (15.7 mL, 90 mmol) were added. The resulting mixture was heated to reflux for 3h. Once this time has elapsed, the solvent was eliminated at low pressure, water (150 mL) was added and the precipitate filtered and washed with methanol to obtain (2-Chloro-9*H*-purin-6-yl)-(6,6-diphenyl-hex-5-enyl)-amine (31.76g, (78.6 mmol, 87.3% yield) as a white solid (m.p.: 205.7°-207.0°C ¹H-NMR (CDCl₃), δ (ppm): 8.1 (s, 2H); 7.5-7.0 (m, 10H); 6.1 (t,1H); 2.5 (m, 2H); 2.1 (m, 2H); 1.7-1.4 (m, 4H).
   The following compounds were prepared in an analogous way, by reaction of 6,6-diphenyl-hex-5-enylamine with the corresponding commercial 6-chloropurine derivatives or 4-chloro-1*H*-pyrazolo[3,4-*d*]pyrimidine derivatives. Non-commercial compounds were prepared by known literature methods such as those described in US6936713; Eur. J. Med. Chem, 2003, 38, 199-214; WO2005/009348; Synthesis, 1994, 1401; J. Am. Chem. Soc. 1956, 78, 784-790; J. Med. Chem. 2005, 6887; J. Med. Chem. 2006, 1549; and/or Eur. J. Med. Chem, 2004, 939.
**Example 30: (9-Benzyl-2-iodo-9*H*-purin-6-yl)-(6,6-diphenyl-hex-5-enyl)amine hydrochloric salt (Compound 30).** The reaction with **6-chloro-2-iodo-9*H*-purine** yielded the title compound. ¹H-NMR (CDCl₃), δ (ppm): 7.5 (s, 1H); 7.4-7.0 (m, 15H); 6.1 (t, 1H); 5.7 (s,1H); 5.3 (s, 2H); 3.6 (s, 2H); 2.1 (q, 2H); 1.6 (m, 5H).
**Example 31: 9-Benzyl-*N*-(6,6-diphenyl-hex-5-enyl)-9*H*-purine-2,6-diamine (Compound 31)** The reaction with 9-benzyl-6-chloro-9*H*-purin-2-ylamine yielded the title compound. ¹H-NMR (CDCl₃), δ (ppm): 7,4-7.1 (m, 15H), 6.1 (t, 1H), 5.5 (m, 1H, NH); 5.2 (s, 2H); 4.7 (s, 2H, NH₂); 3.5 (m, 2H); 2.2 (m, 2H); 1.6 (m, 4H).
**Example 32: (6,6-Diphenyl-hex-5-enyl)-(2-phenyl-9*H*-purin-6-yl)amine (Compound 32)** The reaction with 6-chloro-2-phenyl-9*H*-purine yielded the title compound. ¹H-NMR (CDCl₃+ DMSO-*d*₆), δ(ppm): 8.2 (m, 2H); 7.7 (s, 1H); 7.4-7.0 (m, 14H); 6.9 (bs, 1H); 6.0 (t, 1H); 3.6 (m, 2H); 2.1 (q, 2H); 1.8- 1.4 (m, 4H).
**Example 33: (6,6-Diphenyl-hex-5-enyl)-(9-methyl-2-trifluoromethyl-9*H*-purin-6-yl)amine (Compound 33)** The reaction with 6-chloro-9-methyl-2-trifluoromethyl-9*H* purine yielded the title compound. ¹H-NMR (CDCl₃), δ (ppm): 7.8 (s,1H); 7.4-7.0 (m, 10H); 6.1 (t, 1H); 5.8 (bs, 1H); 3.9 (s, 3H); 3.6 (bs, 2H); 2.2 (q, 2H); 1.8-1.5 (m, 4H).
**Example 34: (5,5-Diphenyl-pent-4-enyl)-(9-methyl-9*H*-purin-6-yl)amine (Compound 34)** The reaction with 6-chloro-9-methyl-9H-purine yielded the title compound.¹H-NMR (CDCl₃), δ (ppm): 8.4 (s, 1H); 7.7 (s, 1H); 7.4-7.0 (m, 10H); 6.1 (t, 1H); 5.7 (s, 1H, NH); 3.9 (s, 3H); 3.6 (m, 2H); 2.3 (q, 2H); 1.9 (m, 2H).
**Example 35 : Synthesis of (2-Chloro-9-methyl-9*H*-purin-6-yl)-(6,6-diphenyl-hex-5-enyl)amine (Compound 35)** 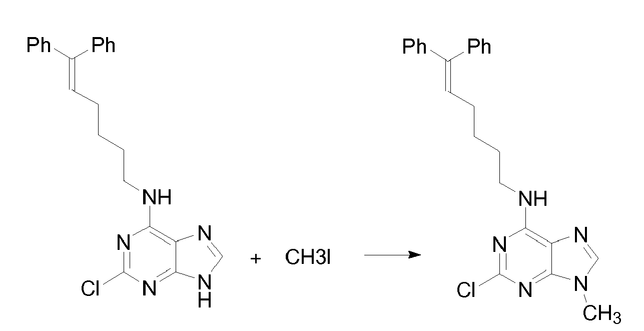 To a solution of (2-chloro-9*H*-purin-6-yl)-(6,6-diphenyl-hex-5-enyl)-amine (2.0 g, 5.0 mmol) in DMF (50 mL), potassium carbonate (1.4 g, 10 mmol) was added, and the resulting suspension was treated with methyl iodide (0.5 mL, 7.92 mmol). The reaction mixture was stirred at ambient temperature for 72 h. Water (250 mL) was added and the resulting mixture was extracted with ethyl acetate (3 x 50 mL). The organic extract was washed with a brine (1 x 25 mL), dried over anhydrous sodium sulfate and the solvent was eliminated at low pressure, to obtain a solid residue (2.30 g), which was purified by flash chromatography (EtOAc/Heptane 60:30) to give 2-chloro-9-methyl-9*H*-purin-6-yl)-(6,6-diphenyl-hex-5-enyl)amine (1.45 g, 70 % yield) as a white solid ¹H-NMR (CDCl₃), δ (ppm): 7.7 (s, 1H); 7.4-7.0 (m, 10H); 6.1 (t, 1H); 5.9 (bs, 1H); 3.8 (s, 3H); 3.6 (bs, 2H); 2.2 (q, 2H); 1.7-1.5 (2q, 4H).
**Example 36 : Synthesis of (9-Benzyl-2-methylsulfanyl-9*H*-purin-6-yl)-(6,6-diphenyl-hex-5-enyl)amine (Compound 36)** 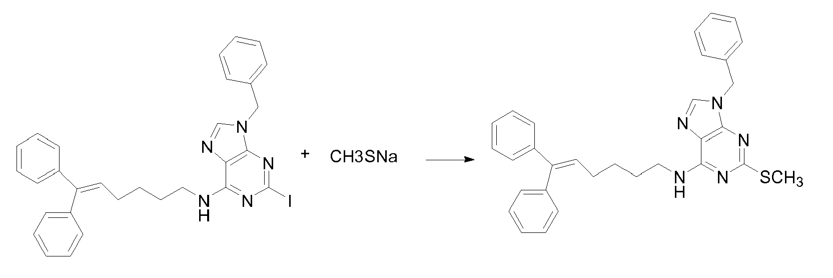 In a 250 mL high pressure reactor, a solution of (9-benzyl-2-iodo-9*H*-purin-6-yl)-(6,6-diphenyl-hex-5-enyl)-amine (compound 30) (2.4 g, 4.1 mmol in a mixture of ethanol (30 mL) and water (30 mL), was treated with sodium thiomethoxide (1.4 g, 20.5 mmol). Reactor vessel was closed and the mixture heated at 130°C for 6 h. (maximum internal pressure: 2 bar.) Once this time elapsed, the solvent was removed at low pressure, water (120 mL) was added and the resulting mixture was extracted with ethyl acetate (3 x 50 mL). The organic layers were dried over anhydrous sodium sulphate and the solvent was evaporated under vacuum to give a residue, which was purified by flash chromatography (EtOAc/Heptane 1:1) to give (9-benzyl-2-methylsulfanyl-9*H*-purin-6-yl)-(6,6-diphenyl-hex-5-enyl)amine (1.68 g, 3.3 mmol, 81%) as a yellow solid. ¹H-NMR (CDCl₃), δ (ppm): 7.55 (s, 1H); 7.45-7.12 (m, 15H); 6.07 (1H, t); 5.65 (bs, 1H); 5.29 (s, 2H); 3.58 (bs, 2H); 2.55 (s, 3H); 2.16 (q, 2H); 1.79-1.51 (m, 4H).
**Example 37: (6,6-Diphenyl-hex-5-enyl)-(9-methyl-2-phenyl-9*H*-purin-6-yl)amine (Compound 37)** 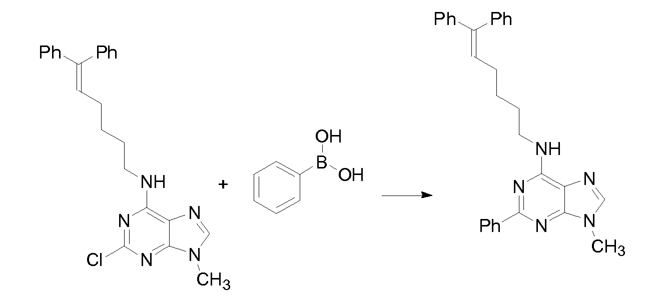 A suspension of 2-chloro-9-methyl-9*H*-purin-6-yl)-(6,6-diphenyl-hex-5-enyl)amine (3.0 g, 7.0 mmol), sodium carbonate (1.3 g, 12.28 mmol), phenyl boronic acid (1.05 g, 8.6 mmol) and tetrakis(triphenylphosphine palladium (0) (1.21 g, 1.05 mmol, 15 mol %) in DME (50 mL) was stirred at 100 °C for 40h under Ar.
   Once this time elapsed, the solvent was removed at low pressure and water (34 mL) was added. The resulting mixture was stirred for 10 min. and the precipitate filtered and washed with water (2 x 20 mL) and ether (2 x 20 mL). The so-obtained material was dried under vacuum yielding 2-chloro-9-methyl-9*H*-purin-6-yl)-(6,6-diphenyl-hex-5-enyl)amine (2.6 g, 5.7 mmol, 81 % yield) as a yellow solid. ¹H-NMR (CDCl₃), δ(ppm): 8.5 (d, 2H); 7.7 (s, 1H); 7.5-7.1 (m, 13H); 6.1 (t, 1H); 5.7 (m, 1H, NH); 3.9 (s, 3H); 3.7 (m, 2H); 2.3 (q, 2H); 1.8 (m, 2H); 1,6 (q, 2H).
   The following compounds were prepared in an analogous way, by reaction of 2-chloro-9-methyl-9*H*-purin-6-yl)-(6,6-diphenyl-hex-5-enyl)amine with the corresponding heteroaryl boronic acids derivatives:
**Example 38: (6,6-Diphenyl-hex-5-enyl)-(9-methyl-2-thiophen-2-yl-9*H*-purin-6-yl)amine (Compound 38)** ¹H-NMR (CDCl₃), δ(ppm): 7.7-7.5 (m, 2H); 7.5-7.0 (m, 11H); 6.1 (t, 1H); 5.4 (bs, 1H); 3.7 (s, 3H); 3.6 (bs, 2H); 2.1(q, 2H); 1.8-1.5 (2q, 4H).
**Example 39: (6,6-Diphenyl-hex-5-enyl)-(9-methyl-2-pyridin-4-yl-9*H*-purin-6-yl)amine (Compound 39**) ¹H-NMR (CDCl₃, δ(ppm): 8.7 (d, 2H); 8.3 (d, 2H); 7.7 (s, 1H); 7.3-7.0 (m, 10H); 6.1 (t, 1H); 5,5 (bs, 1H); 3.9 (s, 3H); 3.7 (bs, 2H); 2.2 (q, 2H); 1.8-1.5 (2q, 4H).
**Example 40: (6,6-Diphenyl-hex-5-enyl)-(1*H*-pyrazolo[3,4-*d*]pyrimidin-4-yl)amine. (Compound 40)** ¹H-NMR (CDCl₃, DMSO-*d*₆), δ (ppm): 12.81 (bs, 1H); 8.22 (s, 1H); 7.92 (s, 1H); 7.02-7.26 (m, 10H); 6.80 (bs, 1H); 5.95 (t, 1H); 3.41-3.45 (m, 2H); 2.45-2.58 (m, 2H); 2.03-2.10 (m, 2H); 1.42-1.62 (m, 4H).
**Example 41: (5,5-Diphenyl-pent-4-enyl)-(1*H*-pyrazolo[3,4-*d*]pyrimidin-4-yl)amine (Compound 41)**
   . ¹H-NMR (DMSO-*d*₆), δ (ppm): 13.35 (bs, 1H); 8.11 (bs, 1H); 8.04 (s, 1H); 7.05-7.33 (m, 10H); 6.16 (t, 1H); 3.42-3.49 (m, 2H); 2.08-2.15 (m, 2H); 1.72-1.77 (m, 2H);
   The following compounds were prepared in an analogous way that compound 35, by reaction of compound 41 with the corresponding alkyl benzyl halides derivatives.
**Example 42: (6,6-Diphenyl-hex-5-enyl)-(1-methyl-1*H*-pyrazolo[3,4-*d*]pyrimidin-4-yl)amine hydrochloric salt. (Compound 42)** ¹H-NMR (CDCl₃), δ (ppm): 8.35 (bs, 1H); 7.83 (s, 1H);. 7.14-7.39 (m, 10H); 6.07 (t, 1H); 4.04 (s, 3H); 3.5-3.6 (m, 2H); 2.19 (q, 2H); 1.67-1.75 (m, 2H); 1.56-1.63 (m, 2H).
**Example 43: (1-Benzyl-1*H*-pyrazolo[3,4-*d*]pyrimidin-4-yl)-(6,6-diphenyl-hex-5-enyl)amine hydrochloric salt (Compound 43)** ¹H-NMR (CDCl₃), δ (ppm): 8.4 (bs, 1H); 7.9 (s, 1H); 7.4-7.2 (m, 16H); 6.1 (t, 1H); 5.6 (s, 2H); 3.6-3.5 (m, 2H); 2.2 (q, 2H); 1.9-1.8 (m, 2H); 1.6-1.5 (m, 2H).
**Example 44: (6,6-Diphenyl-hex-5-enyl)-(1-isopropyl-1*H*-pyrazolo[3,4-*d*]pyrimidin-4-yl)amine hydrochloric salt. (Compound 44)** ¹H-NMR (DMSO-*d*₆), δ (ppm): 11.0 (bs, 1H); 8.8 (s, 1H); 8.4 (s, 1H); 7.3-7.1 (m, 10H); 6.1 (t, 1H); 5.0 (hept, 1H); 3.7-3.4 (m, 2H); 2.1-1.9 (m, 2H); 1.7-1.6 (m, 2H); 1.6-1.40 (m, 2H); 1.39 (d, 6H).

### BIOLOGICAL ASSAYS

### Generation of transfectants.

All transfectants had a common cellular base devoid of those key receptors of the routes under study. The secreted phosphatase sPase was used as reporter for its ability to process the luminiscent or colorimetric substrates from the culture based in the hydrolysis of substrates chemically similar to nitrophenol phosphate. Five generation of transfectants were developed as described:
1. IL-4R. Chains of Type II IL-4r, which is the common one within non-haematopoietic cells were transfected, as well as STAT6 which the key molecule in the nuclear signalling route by IL-4r, and finally a reporter gene to measure the response to STAT6 was introduced. Endogenously, cells had Janus Associated Kinase 1 (JAK1) which was previously known to be associated with STAT6 via p62/aPKC.
2. IL-6R. Chains of IL-6R were transfected and a reporter gene able to measure the response to STAT3 was introduced. As it is well known, STAT3 is a transductional molecule and a nuclear factor physiologically produced in the standard responses IL-6/IL-6R.
3. TNF-R. In this case, a reporter gene able to measure the response to NF-kB was introduced, as this is the transductional molecule and the nuclear factor physiologically produced in response to TNF-alpha. The TNF-alpha transfectants did not respond to potent activators ofNFkB as LPS.
4. LPS-Receptor complex represented by TLR4/MD2/CD14. Reporter genes with elements of response to NFkB were used as in the previous case, but the selectivity was achieved by the co-transfection of components of the LPS-R complex. The LPS is captured from the serum through the union to one plasma protein known as LBP, which transfers the LPS to the membrane protein CD14. The LBP inclusion accounts for an increase of 1000-fold the sensitivity versus media or cultures LBP-frees. The molecule CD14 is not an LPS-receptor but a module of the receptor complex which captures LBP, allowing the transference of LPS in the cellular surface to MD2. CD14 has not a cytoplasmic tail and it is not able to be a signal transducer but it substantially increases the sensitivity of the system. In a similar way, MD2 is not a signal transducer but the combination of CD14 and MD2 allows the increase of the sensitivity by 10000-fold.
5. IL-1R. Reporter genes with elements of response to NFkB were used as in the previous case, but the selectivity was achieved by the expression of components belonging to the receptor complex IL-1R. IL-1R belongs to a receptors superfamily which uses TIR transduction domains (Toll-like IL-1R). It is known that the IL-1R route activates mitogen-activated protein kinases (MAPK) of JNK type which in turn generate nuclear factors transcriptionally active as AP-1. The reporter gene includes NFkB and AP-1 sites chained in order to simulate the physiological situation.

| **Compound** | **Results** | |
|---|---|---|
| Compound 28 | Inhibition of TNFr | **25%** |
| | Inhibition of IL6r | **down-low (*)** |
| | Inhibition of IL4r | **75%** |
| Compound 27 | Inhibition of TNFr | **total** |
| | Inhibition of IL6r | **low-75%** |
| | Inhibition of IL4r | **total** |
| Compound 13 | Inhibition of TNFr | **total** |
| | Inhibition of IL6r | **total** |
| | Inhibition of IL4r | **total** |
| Compound 11 | Inhibition of TNFr | **total** |
| | Inhibition of IL6r | **total** |
| | Inhibition of IL4r | **total** |
| Compound 10 | Inhibition of TNFr | **up 30%** |
| | Inhibition of IL6r | **down 5%** |
| | Inhibition of IL4r | **total** |
| Compound 8 | Inhibition of TNFr | **total** |
| | Inhibition of IL6r | **total** |
| | Inhibition of IL4r | **total** |
| Compound 6 | Inhibition of TNFr | **total** |
| | Inhibition of IL6r | **total** |
| | Inhibition of IL4r | **total** |

### Murine Endotoxemia: Activity of Compound 11 and Compound 8 on Blood Serum Levels of TNF-α.

**MATERIAL AND METHODS:** Male CD1 mice, weighing between 30 and 35 g and housed under controlled standard conditions, were used. These animal fasted (for 14-16 hours; 12 per group; 6 per cage ≅ 500 cm²), with a nutritional solution made of Saccharose (8 %) and NaCl (0.2 %), *ad libitum.* Oral test treatments were given in suspensions (vehicle: Tween 80 0.1 % - NaCl 0.9 %), in a 10 ml/kg volume ratio. One hour later, murine endotoxemia was induced by intraperitoneal injection of Lipopolysaccharides from *Escherichia coli* serotype 055:B5 (Sigma L-2880), at 1 mg/kg dose and in a 10 ml/kg volume ratio, dissolved in sterile saline. Ninety minutes after LPS injection (i.p.), under Isoflurane anaesthesia, 0.5-0.8 ml of blood was extracted by cardiac punction; the blood sample was centrifuged (6000 rpm; 10 minutes; 4°C) and 2 serum samples were taken and kept frozen at -70°C until serum TNF-α concentrations were measured (EIA: Mouse TNF/TNFSF1A Quantikine from R&D Systems).

The compounds 11 and 8, given orally at 100 mg/kg/10ml doses, show activity in this experimental model of LPS (i.p.) from *E.coli* induced murine endotoxemia; they reduce 35.42% and 37.7% respectively, in a statistically significant way, blood serum levels of TNF-α, in relation to its vehicle group.

### Blood Serum levels of IL6 in mice treated with parathyroid hormone. Activity of Compound 15.

**MATERIAL AND METHODS**: Male CD1 mice, between 5 and 7 week and housed under controlled standard conditions, were used. These animal fasted (for 14-16 hours; 12 per group; 6 per cage ≅ 1100 cm²), with solid and liquid food *ad libitum.*

During 5 days, the animals were treated with 8 i.p. administration of pTH-rp (1-34, human) 15 micro gram/0.1 ml/injection

Oral test treatments were given in suspensions (vehicle: Tween 80 0.1 % - NaCl 0.9 %), in a 10 ml/kg volume ratio., one hour later of pTH injection.

Two hours after last pTH administration and under Isoflurane anaesthesia, 0.5-0.8 ml of blood was extracted by cardiac punction; the blood sample was centrifuged (6000 rpm; 10 minutes; 4°C) and 2 serum samples were taken and kept frozen at -70°C until serum IL6 concentrations were measured (EIA: reference number 555240. BD Bioscience).

The compounds 15, given orally at 25 mg/kg/10ml doses, show activity in this experimental model; it reduces 64.58%, in a statistically significant way, blood serum levels of IL6, in relation to its vehicle group.

### ANTITUMORAL ACTIVITY OF COMPOUND 3.

### CELL LINE NCI PANNEL (PRIMARY SCREENING)

The distinct human cell lines used in this study were obtained from the American Type Culture Collection, and were cultured in RPMI-1640 medium containing 10% (v/v) heat-inactivated fetal bovine serum, 2 mM L-glutamine, 100 U/ml penicillin, and 100 µg/ml streptomycin at 37°C in humidified 95% air and 5% CO₂.

Distinct amounts of exponentially growing human cell lines were seeded in 96-well flat-bottomed microtiter plates in a final volume of 100 µl, and incubated at 37°C in a humidified atmosphere of 5% CO₂/95% air for 24 h to allow the cells attach to the plates. Then, cells were incubated with different concentrations of the assayed compound at 37°C under the 5% CO₂/95% air atmosphere for 72 h. Cell proliferation was quantified using the XTT (3'-[1-(phenylamino)carbonyl]-3,4-tetrazolium-bis(4-methoxy-6-nitro)benzene sulfonic acid sodium salt hydrate) cell proliferation kit (Roche Molecular Biochemicals, Mannheim, Germany) following the manufacturer's instructions. Briefly, a freshly prepared mixture solution (50 µl) of XTT labeling reagent and PMS (*N*-methyldibenzopyrazine methyl sulfate) electron coupling reagent was added to each well. The resulting mixtures were further incubated for 4 h in a humidified atmosphere (37°C, 5% CO₂), and the absorbance of the formazan product generated was measured with a microtiter plate reader at a test wavelength of 490 nm and a reference wavelength of 655 nm. The IC₅₀ (50% inhibitory concentration) was then calculated as the drug concentration causing a 50% inhibition of cell proliferation. Data are shown as mean values (± S.D.) of four independent experiments each performed in triplicates. Table 1 shows some of the IC50 concentrations for the tested compounds after the screening in both 3 and 60 cell-line panels.

**Table 1. IC50 (micromolar) in cell-lines screening**

| **Cell line** | **Tissue** | **IC50 (Molar)** |
|---|---|---|
| BT-549 | Breast | 1.6 ± 0.01 E-05 |
| HS 578T | Breast | 1.1 ± 0.08 E-05 |
| MCF-7 | Breast | 1.3 ± 0.08 E-05 |
| MDA-MB-231 | Breast | 1.3 ± 0.11 E-05 |
| MDA-MB-435 | Breast | 1.5 ± 0.41 E-05 |
| MDA-MB-468 | Breast | 8.7 ± 0.01 E-06 |
| NCI/ADR-RES | Breast | 1.4 ± 0.08 E-05 |
| SK-BR-3 | Breast | 1.2 ± 0.25 E-05 |
| T-47D | Breast | 1.1 ± 0.29 E-05 |
| SF-268 | CNS | 8.9 ± 0.42 E-06 |
| SF-295 | CNS | 8.1 ± 0.15 E-06 |
| SF-539 | CNS | 6.6 ± 0.32 E-06 |
| SNB-19 | CNS | 1.5 ± 0.05 E-05 |
| SNB-75 | CNS | 1.5 ± 0.08 E-05 |
| U251 | CNS | 1.6 ± 0.01 E-05 |
| COLO-205 | Colon | 1.3 ± 0.11 E-05 |
| HCT-15 | Colon | 4.8 ± 2.10 E-06 |
| HCT-116 | Colon | 6.0 ± 1.10 E-06 |
| HT-29 | Colon | 1.5 ± 0.12 E-05 |
| KM12 | Colon | 1.6 ± 0.05 E-05 |
| SW-620 | Colon | 8.8 ± 0.74 E-06 |
| CCRF-CEM | Leukemia | 6.1 ± 0.04 E-06 |
| HL-60 | Leukemia | 1.2 ± 0.05 E-05 |
| K-562 | Leukemia | 4.6 ± 1.60 E-06 |
| MOLT-4 | Leukemia | 1.5 ± 0.07 E-05 |
| LOX IMVI | Melanoma | 1.1 ± 0.04 E-05 |
| M14 | Melanoma | 7.5 ± 0.30 E-06 |
| MALME-3M | Melanoma | 8.7 ± 0.33 E-06 |
| SK-MEL-2 | Melanoma | 1.4 ± 0.04 E-05 |
| SK-MEL-5 | Melanoma | 1.4 ± 0.20 E-05 |
| SK-MEL-28 | Melanoma | 1.2 ± 0.11 E-05 |
| UACC-62 | Melanoma | 1.6 ± 0.05 E-05 |
| UACC-257 | Melanoma | 3.7 ± 0.07 E-06 |
| RPMI-8226 | MM | 5.9 ± 0.67 E-06 |
| A549 | Non-Small Cell Lung | 1.3 ± 0.18 E-05 |
| EKVX | Non-Small Cell Lung | 1.6 ± 0.04 E-05 |
| HOP-62 | Non-Small Cell Lung | 1.3 ± 0.11 E-05 |
| HOP-92 | Non-Small Cell Lung | 6.2 ± 0.55 E-06 |
| NCI-H23 | Non-Small Cell Lung | 8.8 ± 0.56 E-06 |
| NCI-H322M | Non-Small Cell Lung | 1.1 ± 0.04 E-05 |
| NCI-H226 | Non-Small Cell Lung | 8.7 ± 0.27 E-06 |
| NCI-H460 | Non-Small Cell Lung | 1.3 ± 0.15 E-05 |
| NCI-H522 | Non-Small Cell Lung | 1.1 ± 0.08 E-05 |
| IGR-OV1 | Ovarian | 6.2 ± 0.19 E-06 |
| OVCAR-3 | Ovarian | 1.2 ± 0.17 E-05 |
| OVCAR-5 | Ovarian | 6.5 ± 0.27 E-06 |
| OVCAR-8 | Ovarian | 1.5 ± 0.08 E-05 |
| SK-OV-3 | Ovarian | 1.2 ± 0.12 E-05 |
| DU-145 | Prostate | 1.3 ± 0.16 E-05 |
| PC-3 | Prostate | 1.4 ± 0.08 E-05 |
| 786-0 | Renal | 2.6 ± 0.22 E-05 |
| A498 | Renal | 3.3 ± 0.11 E-05 |
| ACHN | Renal | 1.5 ± 0.01 E-05 |
| CAKI-1 | Renal | 1.2 ± 0.12 E-05 |
| SN12C | Renal | 6.7 ± 0.17 E-06 |
| TK-10 | Renal | 1.5 ± 0.04 E-05 |
| UO-31 | Renal | 1.3 ± 0.15 E-05 |
| DMS 114 | Small Cell Lung | 1.5 ± 0.13 E-05 |
| SHP-77 | Small Cell Lung | 8.1 ± 0.88 E-06 |

HUMAN NON-TUMORAL CELLS (SECUNDARY SCREENING)

The cytotoxicity of this compound for human non-tumoral cells was studied in fibroblasts, HUVEC cells and PBLs (Peripheral Blood lymphocytes).

HUVEC cells were obtained by treating human cords with collagenase type I, 01 % in Hank's medium incubating 20 minutes at 37 °C. Cells were collected afterwards and cultivated in medium M199 with supplements of 20 % FBS, 1 % P/S and 25 mg/500 ml of medium ECGF, Cells were grown on a 0.2 % gelatin matrix.

To determine the cytotoxicity was used XTT, yellow tetrazolium salt which is metabolised by active cells to produce the orange formazan dye, directly correlated with the increase of mithocondrial activity and the increase of the number of feasible cells. IC₅₀ was calculated in a triplicate experiment being n=4 in each case. The results are shown in Table 2.

**Table 2. IC50 for human non-tumoral cells**

| **Human non tumoral cells** | **IC50 (Molar)** |
|---|---|
| FIBROBLAST | >10 E-06 |
| HUVEC | 10 E-06 |
| PBLs | 100 E-06 |

### IN VIVO ANTITUMOR ACTIVITY OF COMPOUND 3 IN A CHRONIC LYMPHOCYTIC LEUKAEMIA XENOGRAFT ANIMAL MODEL.

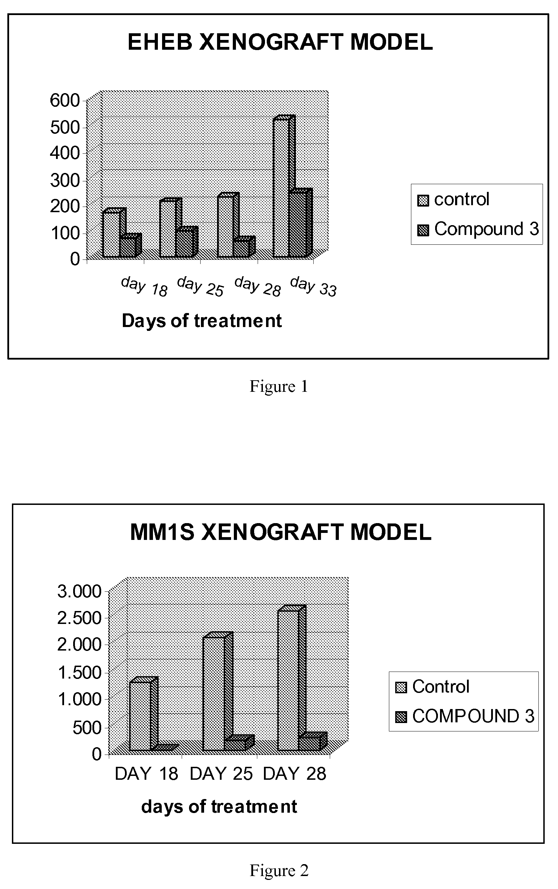

The chronic lymphocytic leukaemia (CLL) EHEB cell line was cultured in RPMI-1640 medium containing 10% (v/v) heat-inactivated fetal bovine serum, 2 mM L-glutamine, 100 U/ml penicillin, and 100 µg/ml streptomycin at 37°C in humidified 95% air and 5% CO₂. Cells were periodically tested for *Mycoplasma* infection and found to be negative. CB17-severe combined immunodeficiency (SCID) mice (Charles River laboratories, Lyon, France), kept and handled according to institutional guidelines, complying with Spanish legislation under a 12/12 h light/dark cycle at a temperature of 22°C, received a standard diet and acidified water *ad libitum.* CB17-SCID mice were inoculated subcutaneously into their lower dorsum with 1.5 x 10⁷ EHEB cells in 100 µl PBS and 100 µl Matrigel basement membrane matrix (Becton Dickinson). When tumors were palpable, mice were randomly assigned into cohorts of 10 mice each, receiving *i.v*. administration (3 times/week) of the compound at the tail. The volume used for each *i.v*. administration was 200 µl, rendering a final drug concentration of 2.5 mg/kg (body weight). Mice *i.v*. administered with an equal volume of vehicle were run in parallel. The shortest and longest diameter of the tumor were measured with calipers, and tumor volume (10⁻³cm³) was calculated using the following standard formula: (the shortest diameter)² × (the longest diameter) × 0.5. Animals were sacrificed, according to institutional guidelines, when the diameter of their tumors reached 3 cm or when significant toxicity was observed. Animal body weight and any sign of morbidity were monitored. Drug treatment lasted for 41 days. Then, tumor xenografts were isolated, measured and weighed, and a necropsy analysis involving tumors and distinct organs was carried out. All values were expressed as means ± SD. Statistical differences between groups were assessed using the Mann-Whitney test or the Student's *t*-test. A *p* value of less than 0.05 was considered statistically significant. Results are depicted in **Fig. 1****.** Compounds 3 significantly (**, *p* < 0.01) inhibited CLL tumor growth compared with the control group (treated with vehicle).

After the completion of the *in vivo* assay, control and Compound 3-treated mice were sacrificed, tumor xenografts were isolated and tumor weight and volume were measured, showing a remarkable and significant (*p* < 0.01) reduction in tumor size and weight in Compound 3-treated mice as compared to control untreated mice. Once the mice were killed, a necropsy analysis involving tumors and distinct organs was carried out. No significant events or changes in the distinct organs analyzed were detected after the necropsy analysis. The weight of the mice was similar in both untreated control and treated mice. These data suggest the lack of significant side effects in the treatment of mice with Compound 3. The tumors isolated from Compound 3-treated mice showed very little vascularization as compared to drug-free tumor-bearing control mice, suggesting that Compound 3 may have anti-angiogenic activities that could potentiate their antitumor cytotoxic activities. These results indicate that compound 3 displays a significant and potent *in vivo* antitumor activity in a CLL xenograft mouse model. In addition, treatment of both drugs was well tolerated and no apparent toxicity was detected in necropsy studies.

For all measure days, the difference of compound 3 over the control was stadistically significant (p<0.05)

### IN VIVO ANTITUMOR ACTIVITY OF COMPOUND 3 IN A MULTIPLE MYELOMA XENOGRAFT ANIMAL MODEL.

The multiple myeloma (MM) MMIS cell line was cultured in RPMI-1640 medium containing 10% (v/v) heat-inactivated fetal bovine serum, 2 mM L-glutamine, 100 U/ml penicillin, and 100 µg/ml streptomycin at 37°C in humidified 95% air and 5% CO₂. Cells were periodically tested for *Mycoplasma* infection and found to be negative. CB17-severe combined immunodeficiency (SCID) mice (Charles River laboratories, Lyon, France), kept and handled according to institutional guidelines, complying with Spanish legislation under a 12/12 h light/dark cycle at a temperature of 22°C, received a standard diet and acidified water *ad libitum.* CB17-SCID mice were inoculated subcutaneously into their lower dorsum with 3 x 10⁷ MM1S cells in 100 µl PBS and 100 µl Matrigel basement membrane matrix (Becton Dickinson). When tumors were palpable, mice were randomly assigned into cohorts of 10 mice each, receiving *i.v*. administration (3 times/week) of the compound at the tail. The compound was dissolved and prepared for *i.v*. administration following FAES instructions. The volume used for each *i.v*. administration was 200 µl, rendering a final drug concentration of 2.5 mg/kg (body weight). Mice *i.v*. administered with an equal volume of vehicle were run in parallel. The shortest and longest diameter of the tumor were measured with calipers, and tumor volume (10⁻³cm³) was calculated using the following standard formula: (the shortest diameter)² × (the longest diameter) × 0.5. Animals were sacrificed, according to institutional guidelines, when the diameter of their tumors reached 3 cm or when significant toxicity was observed. Animal body weight and any sign of morbidity were monitored. Drug treatment was for 1 month. Then, tumors were isolated, measured and weighed, and a necropsy analysis involving tumors and distinct organs was carried out. For all measure days, the difference of compound 3 over the control was stadistically significant (p<0.05). Results are shown in Figure 2.

### ANTITUMORAL ACTIVITY OF COMPOUND 3 ON CHRONIC LYMPHOCYTIC LEUKAEMIA CELLS FROM PATIENTS.

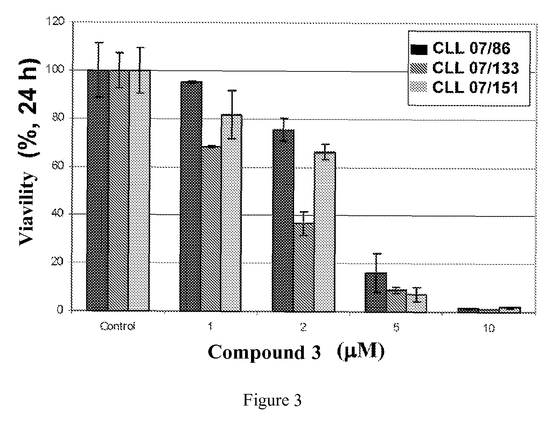

A panel of 6 human patient chronic lymphocytic leukaemia cells was chosen showing different patterns of ZAP70 expression (see cytogenetic changes notated in the table 3). Cells were incubated in the presence of compound 3. Results of this study, showing the viability are depicted in Fig. 3.

**Table 3.**

| **Patient code** | **Diagnostic age** | **sex** | **Estadio** | **%CD19/ CD5** | **%ZAP70** | **Cytogenetic alterations (FISH)** |
|---|---|---|---|---|---|---|
| **CLL 07/86** | 69 | M | B/C | 95 | 70 | normal |
| **CLL 07/133** | 46 | F | B/C | 94 | > 20 | 13q deletion |
| **CLL 07/151** | 54 | M | A | 95 | 19 | normal |

The decrease of viability was more significant in cell with a low expression of ZAP70, in a dose-dependant way.

## Claims

1. A compound of formula (I), or a salt, prodrug or solvate thereof: 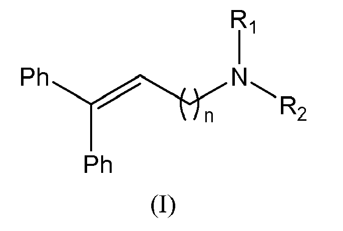 wherein:
Ph is phenyl;
n is 2, 3 or 4;
R₁ is selected from the group consisting of hydrogen and C₁-C₆ alkyl;
R₂ is a radical of formula -[[CH(R₃)]ₘ-R_{4]}, wherein
m is an integer selected from the group consisting of 1, 0 or 2;
each R₃, where appropriate, is selected from the group consisting of hydrogen, phenyl and C₁-C₆ alkyl;
R₄ is selected from the group consisting of an unsubstituted heteroaryl, a substituted heteroaryl and a substituted aryl radical,
said substituents being selected from the group consisting of C₁-C₆ alkyl, C₇-C₁₁ arylalkyl, phenyl, 5- or 6-membered heteroaryl, F, Cl, Br, I, trifluoromethyl, cyano, -N(Rₐ)(R_{b}), -OR_{c}, -SR_{d} or -C(O)Rₑ; wherein Rₐ, R_{b}, R_{c}, R_{d}, and Rₑ are independently selected from hydrogen, C₁-C₆ alkyl, phenyl and trifluoromethyl;
or
if R₁ and/or R₃ are different from hydrogen, then R₄ may also be unsubstituted phenyl;
or
R₁ and R₂, together with the nitrogen atom to which they are attached, form a substituted or unsubustited heteroaryl group, wherein said substituents are as defined above
with the proviso that:
2-[(4,4-diphenyl-but-3-enylamino)-methyl]-phenol,
3-[(4,4-diphenyl-but-3-enylamino)-methyl]-phenol,
5-[(4,4-diphenyl-but-3-enylamino)-methyl]-2-methoxy-phenol, 4-[(4,4-diphenyl-but-3-enylamino)-methyl]-2,6-difluoro-phenol, benzyl-(5,5-diphenyl-pent-4-enyl)-ethyl-amine,
6-Chloro-9-(4,4-diphenyl-but-3-enyl)-9H-purine,
9-(4,4-Diphenyl-but-3-enyl)-9H-purin-6-ylamine, and
5-(4,4-Diphenyl-but-3-enyl)-4,5,6,7-tetrahydro-isoxazolo[4,5-c]pyridin-3-ol
are not included in formula (I).

2. A compound according to claim 1 wherein each of said substituents is selected from the group consisting of C₁-C₆ alkyl, C₇-C₁₁ arylalkyl, phenyl, 5- or 6-membered heteroaryl, F, Br, I, trifluoromethyl, cyano, -N(Rₐ)(R_{b}), -OR_{c}, -SR_{d} or -C(O)Rₑ; wherein Rₐ, R_{b}, R_{d}, and Rₑ are independently selected from hydrogen, C₁-C₆ alkyl, phenyl and trifluoromethyl; and R_{c} is selected from the group consisting of C₁-C₆ alkyl, phenyl and trifluoromethyl, provided that -N(Rₐ)(R_{b}) is not -NH₂.

3. A compound according to any previous claims of formula (IA), or a salt, prodrug and/or solvate thereof 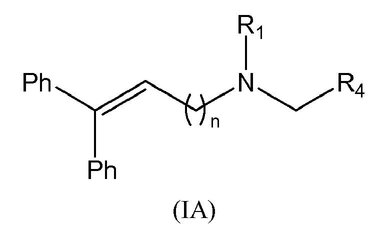 wherein Ph, n, R₁ and R₄ are as defined in any of the previous claims.

4. A compound according to any previous claims wherein R₄ is an unsubstituted heteroaryl or a substituted heteroaryl selected from the group consisting of thienyl, furyl, pyridil, 1*H*-benzimidazol, 9*H*-Purine, 1*H*-Imidazole, and 1*H*-Pyrazolo[3,4-*d*]pyrimidine.

5. A compound according to any of claims 1 to 3, wherein R₄ is a group of formula (V) or (VI) 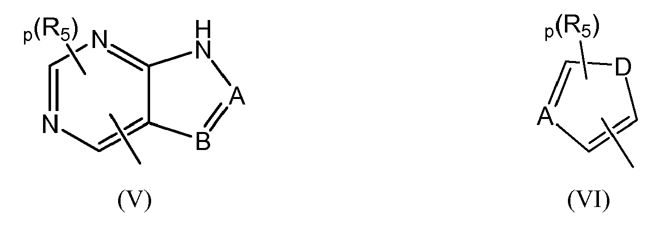 wherein,
A and B are independently selected from -CH- and -N-;
D is independently selected from the group consisting of -O-, -S- and -NH-;
p is an integer selected from the group consisting of 0, 1, 2 or 3;
each R₅ is selected from the group consisting of C₁-C₃ alkyl, phenyl, phenylmethyl, 5- or 6-membered heteroaryl, F, Br, I, trifluoromethyl, -N(Rₐ)(R_{b}), -SR_{d} or - C(O)Rₑ; wherein Rₐ, R_{b}, R_{d}, and Rₑ are independently selected from hydrogen, C₁-C₃ alkyl, phenyl and trifluoromethyl, provided that in a compound of formula (V) - N(Rₐ)(R_{b}) is not -NH_{2;}
said group of formula (V) or (VI) being attached to the rest of the molecule through any of the free positions.

6. A compound according to claim 1 wherein R₁ and R₂, together with the nitrogen atom to which they are attached, form a radical selected from the group consisting of 1*H*-benzimidazol, 9*H*-Purine, 1*H*-Imidazole, and 1*H*-Pyrazolo[3,4-d]pyrimidine.

7. A compound according to claim 1, wherein R₄ is a group of formula (VII) 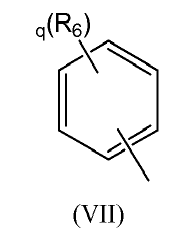 wherein R₆ is selected from the group consisting of -OCF₃, OC₁-C₃alkyl, F, Cl, Br, I and -CN; and q is an integer selected from the group consisting of 1, 2 or 3, and said group of formula (VII) is attached to the rest of the molecule through any of the free positions.

8. Compound according to any of the previous claims, wherein R₁ is hydrogen or methyl.

9. A compound according to claim 1 selected from the group consisting of: 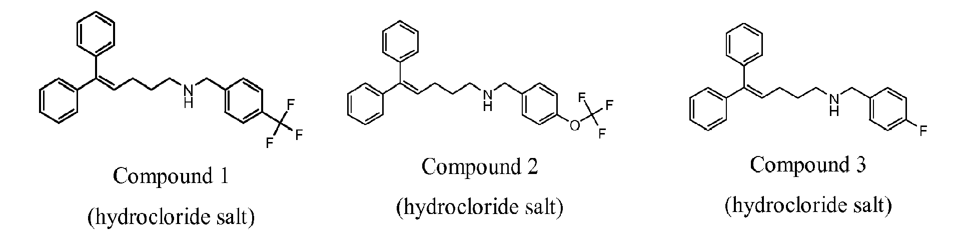 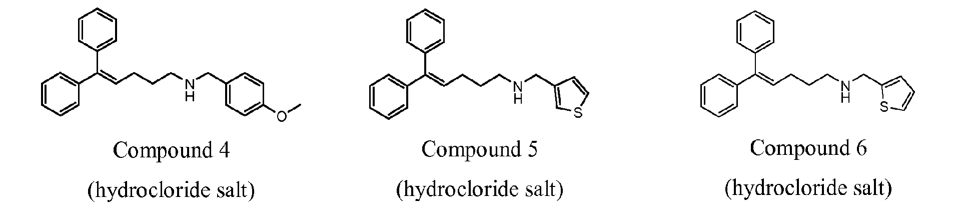 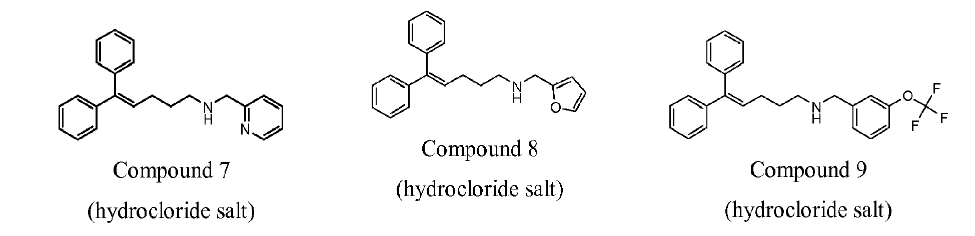 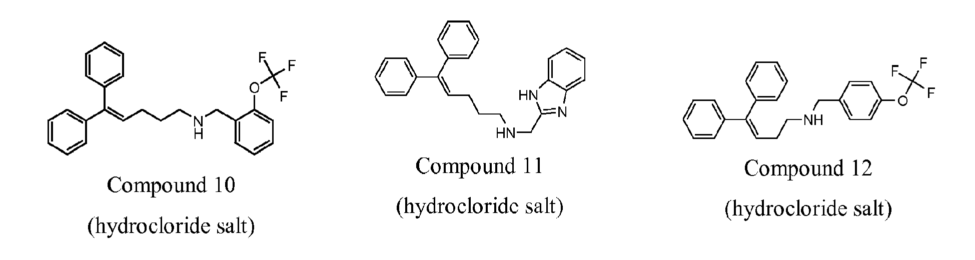 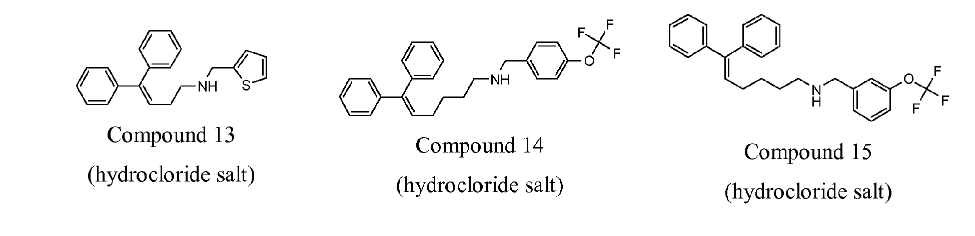 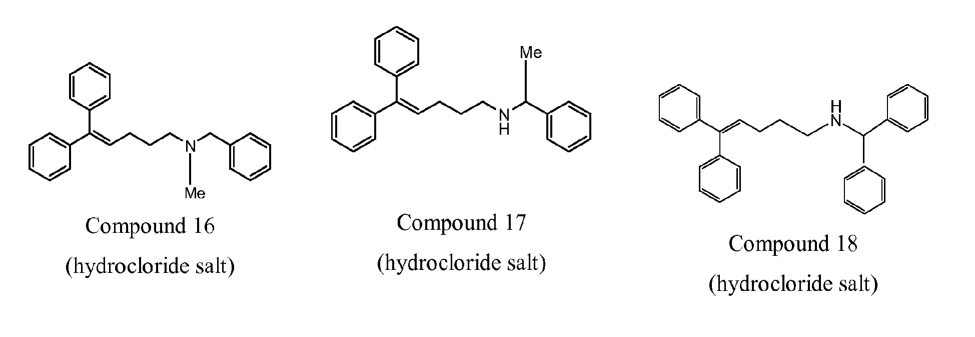 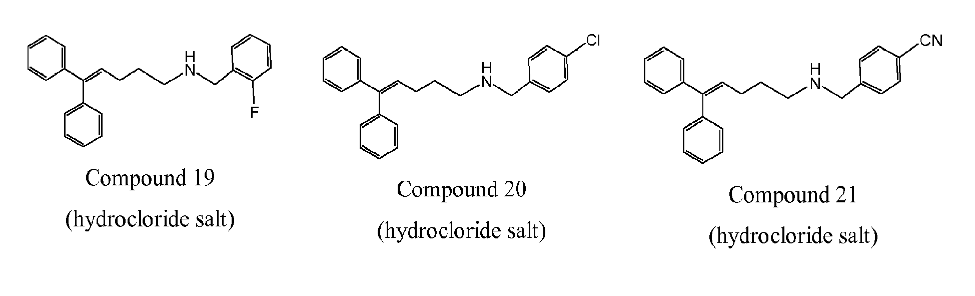 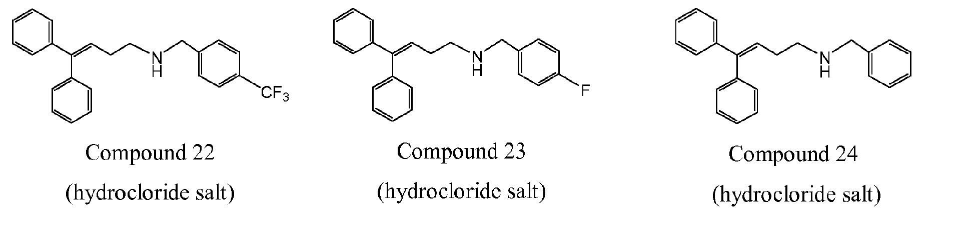 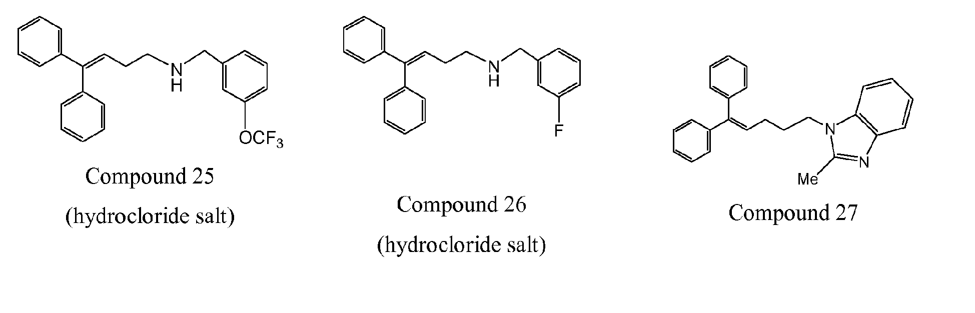 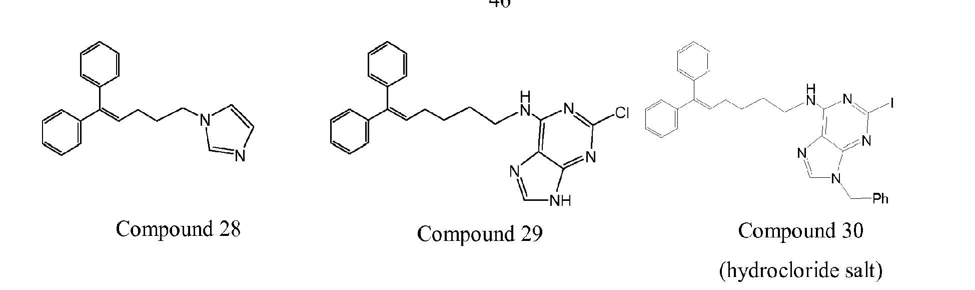 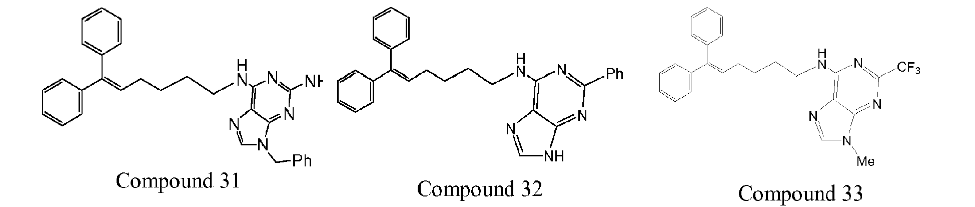 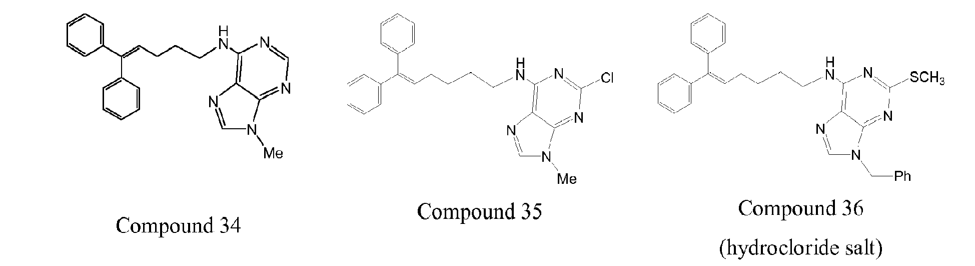 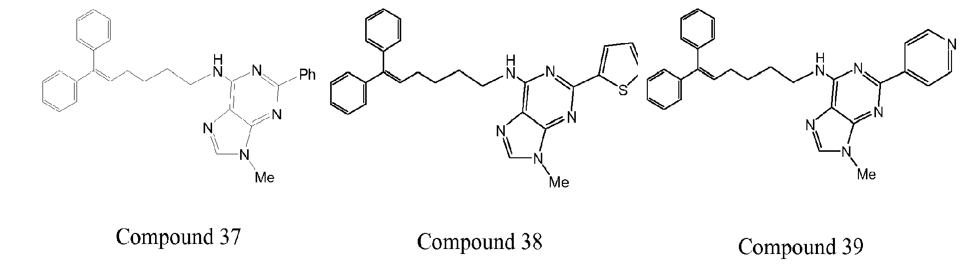 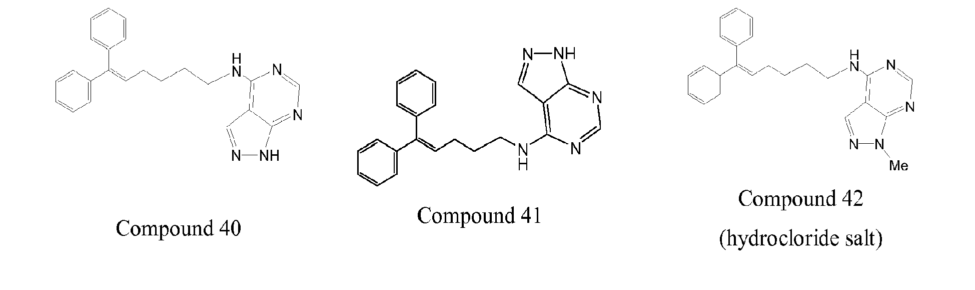 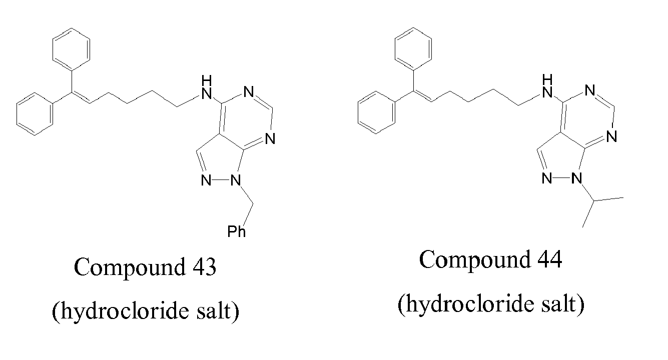 or a salt, prodrug and/or solvate thereof.

10. A pharmaceutical composition comprising a compound of formula (I) as defined in any of claims 1 to 9 or a pharmaceutically acceptable salt, prodrug or solvate thereof, and at least one pharmaceutically acceptable carrier.

11. A compound of formula (I) as defined in any of claims 1 to 9 or a pharmaceutically acceptable salt, prodrug or solvate thereof for use as a medicament.

12. A compound of formula (I), or a pharmaceutically acceptable salt, prodrug or solvate thereof: 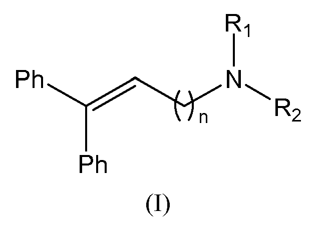 wherein:
Ph is phenyl;
n is 2, 3 or 4;
R₁ is selected from the group consisting of hydrogen and C₁-C₆ alkyl;
R₂ is a radical of formula -[[CH(R₃)]ₘ-R₄], wherein
m is an integer selected from the group consisting of 1, 0 or 2;
each R₃, where appropriate, is selected from the group consisting of hydrogen and C₁-C₆ alkyl;
R₄ is selected from the group consisting of an unsubstituted heteroaryl, a substituted heteroaryl, unsubstitued aryl and a substituted aryl radical,
said substituents being selected from the group consisting of C₁-C₆ alkyl, C₇-C₁₁ arylalkyl, phenyl, 5- or 6-membered heteroaryl, F, Cl, Br, I, trifluoromethyl, cyano, -N(Rₐ)(R_{b}), -OR_{c}, -SR_{d} or -C(O)Rₑ; wherein Rₐ, R_{b}, R_{c}, R_{d}, and Rₑ are independently selected from hydrogen, C₁-C₆ alkyl, phenyl and trifluoromethyl;
or
R₁ and R₂, together with the nitrogen atom to which they are attached, form a substituted or unsubustited heteroaryl group, wherein said substituents are as defined above;
for use as a medicament for treating inflammatory diseases.

13. A compound of formula (I) according to claim 12 wherein the inflammatory disease is selected from the group consisting of Inflammatory Bowel Disease (IBD), Rheumatoid Arthritis (RA), benign prostatic hyperplasia, Barrett's disease, asthma, skeletal muscle and tendon repair, ulcerative colitis, leishmaniasis and autoimmune diseases, preferably Crohn's disease.

14. A compound of formula (I) according to claim 12 for use in the treatment of cancer.

15. A compound according to claim 14 wherein the cancer is selected from the group consisting of metastasis, breast cancer, esophageal cancer, colon cancer, colon carcinomas, stomach cancer, Leukemias, Melanoma, carcinomas of the uterus, non-small cell lung cancer, small cell lung cancer, ovarian cancer, ovarian carcinomas, prostate cancer, renal cancer, liver cancer, carcinomas of the pancreas, kidney cancer, bladder cancer, prostate cancer, testicular cancer, bone cancer, skin cancer, sarcoma, Kaposi sarcomas, brain tumours, myosarcomas, neuroblastomas, limphomas and multiple myeloma.
